# EUROPEAN PATENT SPECIFICATION

(11) **EP 1 689 856 B1**
(45) Date of publication and mention of the grant of the patent: **05.10.2011**
(21) Application number: 04808705.0
(22) Date of filing: 11.11.2004
(51) Int. Cl.: C12N 5/071

(54) **INHIBITION OF STEM CELL DIFFERENTIATION, ENHANCEMENT OF PROLIFERATION AND SELECTIVE INDUCTION OF APOPTOSIS BY WNT FACTORS**
HEMMUNG DER STAMMZELLDIFFERENZIERUNG, VERBESSERUNG DER PROLIFERATION UND SELEKTIVE INDUKTION VON APOPTOSE DURCH WNT-FAKTOREN
INHIBITION DE LA DIFFERENTIATION DES CELLULES SOUCHES, AMELIORATION DE LA PROLIFERATION ET INDUCTION SELECTIVE DE L'APOPTOSE PAR DES FACTEURS WNT

(30) Priority: 11.11.2003 EP 03078549; 11.11.2003 US 519403 P
(43) Date of publication of application: 16.08.2006
(73) Proprietor: DeltaCell B.V., 2333 CK Leiden (NL)
(72) Inventor: KIELMAN, Menno, Frederik, NL-2251 RR Voorschoten (NL)
(74) Representative: van Loon, C.J.J.
(86) International application number: PCT/NL2004/000789
(87) International publication number: WO 2005/052141

(56) References cited:
- WO-A-01/42425
- WO-A-02/055109
- WILLERT KARL ET AL: "Wnt proteins are lipid-modified and can act as stem cell growth factors." NATURE. 22 MAY 2003, vol. 423, no. 6938, 22 May 2003 (2003-05-22), pages 448-452, XP002279471 ISSN: 0028-0836
- SAITOH T ET AL: "Molecular cloning and characterization of WNT3A and WNT14 clustered in human chromosome 1q42 region" BIOCHEMICAL AND BIOPHYSICAL RESEARCH COMMUNICATIONS, ACADEMIC PRESS, SAN DIEGO, CA, US, vol. 284, no. 5, 29 June 2001 (2001-06-29), pages 1168-1175, XP002221427 ISSN: 0006-291X
- JAMORA COLIN ET AL: "Links between signal transduction, transcription and adhesion in epithelial bud development." NATURE (LONDON), vol. 422, no. 6929, 20 March 2003 (2003-03-20), pages 317-322, XP002312969 ISSN: 0028-0836
- OTTO W R: "LUNG STEM CELLS" INTERNATIONAL JOURNAL OF EXPERIMENTAL PATHOLOGY, BLACKWELL SCIENTIFIC, OXFORD, GB, vol. 78, 1997, pages 291-310, XP002942524 ISSN: 0959-9673

## Description

The invention relates to the field of medicine, more in particular to the field of lung medicine. The invention relates to the differentiation of stem cells, in particular to organ tissue regeneration and recovery and to transplantation. The invention more in particular relates to inhibition of stem cell differentiation by manipulating a Wnt pathway. More in particular, the invention relates to enhancing proliferation and controlling the differentiation of lung cells by manipulating a Wnt pathway.

Vital organs like the lung, the kidney, the liver, the pancreas, and the skin are characterized by the presence of organ-specific differentiated epithelial cells based on an organ specific matrix of connective tissue (mesenchymal) cells. The combination of said differentiated cells with the connective tissue matrix is related to the specific function of each such organ. Said specific functions may be as varied as for example gas exchange in the lung, filtration in the kidney, detoxification and conjugation in the liver, insulin production in the pancreatic islet cells or protection against an hazardous environment by the skin. Disease or degeneration of such an organ is often life threatening, because degenerated or lost organ structure is often poorly replaced and because the specialized cells of one organ cannot take over the function of another organ.

Worldwide, solutions have been sought for the treatment of degenerative disease of organs and the skin and its appendices. For the skin, as a relatively simple organ structure, some advances have been made by application of autologous or heterologous skin transplants. Pieces of skin are treated (meshed) in order to be stretched out beyond their former size and cover the skin defects of a patient with meshed skin. Such transplants offer a basis for cells to proliferate and close the skin lesions.

For the specialized organs in the body, regenerating cells within the organ in situ would be most optimal because transplantation is often more difficult and less successful. Transplantation often leads to adverse effects of host versus graft or even graft versus host. It would also be a major achievement if organs or a part thereof, could be kept outside of the body (syn. *ex vivo*) for a period of time, to give them a chance to recover by upregulating the proliferation and differentiation of the organ-specific cells. While the organ is *ex vivo*, the patient is for instance dependent of machines to take over the organ's function. In a later stage, said organ is placed back in the patient. This method is also very suitable for bone marrow cells that are taken out of the patient before treatment of leukemia, and are replaced after the treatment. Said bone marrow cells often are separated in various subpopulations and tested for the absence of leukemic cells to avoid the leukemic cells from reentering the host. Many cells do not survive this *ex vivo* handling; with the result that re-implantation of the bone marrow is less successful.

Especially such highly differentiated cells like for example the kidney cells, the insulin producing cells in the Islets of Langerhans of the pancreas, and glandular and/or hair follicle cells of the dermis, are very difficult to recover, if possible at all, and very difficult to maintain, once taken out of their context in the body.

Many researchers try to find ways of extending the survival time of said specialized cells in culture, and possibilities to proliferate the cells whilst retaining their differentiated state. In practice, it appears to be hard to reach this goal.

In the present invention this objective is reached by enhancement of the Wnt signaling pathway in stem cell/progenitor cells of such an organ comprising differentiated cells. In one embodiment, an extended survival time and proliferation and differentiation and selective enrichment of stem cells of the lung is disclosed.

One of the main potential applications of stem cells is their use in transplantation studies in which these cells reconstitute the cells in the diseased organ. Transplantation is preferably performed with stem cells that can either differentiate in vivo to the desired cell-type or with *in vitro* pre-differentiated stem cells. Said pre-differentiation of stem cells *in vitro* to a desired cell-type is a process that was difficult to control before this invention.

One aspect of the present disclossure provides a method to direct the differentiation of stem cells to a desired cell-type *in vitro* in order to use the differentiated cells for transplantation purposes (see for instance example I). Control of said differentiation is achieved by limiting the enhancement of the Wnt signaling pathway to a certain window in these stem cells. Control of the Wnt-signaling pathway is achieved by the addition of at least one Wnt.3a or Wnt 3a variant having at least 90% amino acid sequence identity with Wnt 3a. Without being taught by theory, it is thought that the fluctuation of the concentration of intracellular beta catenin is kept within a narrow range thereby promoting the differentiation of a limited number of cell-types.

The Wnt gene family encodes developmentally important secreted factors, involved in cell growth, differentiation and organogenesis (Wodartz & Nusse, 1998). Wnt genes encode a family of secreted glycoproteins that modulate cell fate and behavior in embryos through activation of receptor-mediated signaling pathways. Wnt signaling events are initiated by receptor activation involving binding to the cysteine-rich domain (CRD) of frizzled 7-transmembrane receptor protein (Fz) (Bhanot et al., 1996). A classical Wnt signal suppresses the activity of glycogen synthase kinase 3 (GSK-3), leading to changes in phosphorylation and increased stability of the β-catenin protein in the cytoplasm (Hinck et al., 1994). β-catenin is essential for activating target genes in response to Wnt signaling (Miller & Moon, 1996; Willert & Nusse, 1998), since it complexes with HMG box transcription factors of the TCF/LEF family (Behrens et al., 1996; Molenaar et al., 1996; Huber et al., 1996). Wnt sequences, patterns of expression and activities are highly conserved in evolution, so it has been possible to gain insights into the functions, and mechanisms of action, of the Wnt genes through a synthesis of genetic and cell biological approaches in different organisms. These studies suggest that there are functionally distinct WNT proteins as assayed by the ability to transform cells and by differences in embryonic responses to ectopic WNT signals. Moreover, gain-of-function and loss-of-function studies both support the involvement of Wnt proteins in modulating cell fate and cell behavior during vertebrate development, often through combinatorial interactions with other signaling pathways to regulate gene expression (Moon RT, Brown JD, Torres M. Trends Genet 1997 Apr; 13(4):157-62). This is supported by data on the ability and sensitivity of mouse embryonic cells to differentiate into three germ layers, which was inhibited by specific mutations in the adenomatous polyposis coli gene.(Kielman et al., 2002)

Components of the Wnt signaling pathway have been found to be present during organogenesis in the mouse (Roelink & Nusse, 1991; Buhler et al., 1993; Parr et al., 1993; Christiansen et al., 1995; Wang & Shackleford, 1996; Cho & Dressler, 1998; Korinek et al., 1998; Leimester et al., 1998). Moreover, loss of function of Wnt and Wnt-related genes leads to abnormal development in the mouse (McMahon & Bradley, 1990; Monkley et al., 1993; Takada et al., 1994; Stark et al., 1994; Galceran et al., 1999; Liu et al., 1999; Yamaguchi et al., 1999; Brisken et al., 2000; Lee et al., 2000). Wnt signaling is inhibited by the presence of Dickkopf proteins. Said Dickkopf (Dkk) proteins bind to the LRP co-receptor for Wnt. Modulating the Wnt pathway in cell culture can for instance be performed by genetic modification (Kielman et al., 2002) or by administering soluble factors influencing the Wnt pathway. The latter method has been used for monolayers of cells in cell culture wherein the cells had no tissue connection to each other like for example hemopoietic stem cells (Matthews et al., 2000). In organ culture, the effects of soluble factors is not easily predicted or understood, because of the three dimensional structure of the organ and because the microenvironment of the cells is more complex by the presence of other cells such as connective tissue and blood vessels.

The present invention provides the controlling of the canonical Wnt signaling pathway, and discloses Wnt3a functioning as a protein of the canonical Wnt-pathway. In one aspect o the present invention, it is demonstrated that Wnt3a is capable of inhibiting the proteolytic breakdown of intra-cellular beta-catenin, thereby inhibiting and/or modulating the differentiation and replication of pluripotent stem cells in the lung and of omni-potent stem cells.

In describing the present invention, the following terms will be employed, and are defined as indicated below.

Lung tissue in the present invention comprises at least two or more of the following compartments: the alveolar compartment, the bronchiolar compartment, the bronchial compartment and the tracheal compartment. A lung cell is defined herein as an epithelial cell of at least one of these four compartments. A population of lung cells comprises at least two lung cells. In this invention, in a preferred embodiment, a population of lung cells together is capable of functioning in the exchange of gasses between the organism's blood compartment and the atmosphere. In the lung, epithelial cells and supporting tissue is present. The supporting tissue comprises connective tissue, nerves and lymph and blood vessels. In this application, connective tissue is described by the term mesenchym.

Omnipotent stem cells are cells that possess the ability to proliferate in-definitively and posses the capacity to differentiate into any possible cell-type that is present in the full-grown organism.

Pluripotent stem cell are cells that possess the ability to proliferate in-definitively and posses the capacity to differentiate into a large but limited number of cell-types that in general belong to a certain tissue such as the lung or a certain cell lineage such as the endoderm, ectoderm or mesoderm.

Stem cells of the invention are genetically modified or not and/or they are obtained by nuclear transfer or not.

The canonical Wnt-signaling pathway a signaling cascade in which a collection of proteins controls the proteolytic breakdown of beta-catenin. Some proteins in this pathway are chemically modified by other proteins and/or are capable of inducing other molecules such as for instance sugars and lipids that often have an influence on the proteolytic breakdown of beta-catenin.

Wnt3a protein is part of a large family of proteins that have a close resemblance to each other in structure and function. Wnt proteins are strongly conserved during evolution in structure and function and are interchangeable between species. The invention therefore comprises all Wnt proteins or a functional part, derivative or analogue thereof that, like Wnt3a, are capable of inducing the canonical Wnt pathway. Other components of the Wnt signaling pathway or fragments of these components, or compounds mimicking the function of these components, that in the end reduce the proteolytic breakdown of beta-catenin will have the same effect as Wnt3a in kind, not necessarily in amount, and are also within the scope of the present disclosure.

The terms "Wnt" or "*Wnt* polypeptide" when used herein encompass native sequence *Wnt* polypeptides, *Wnt* polypeptide variants, and chimaeric *Wnt* polypeptides. Optionally, the *Wnt* polypeptide is not associated with native glycosylation or palmitolyation. "Native glycosylation" refers to the carbohydrate moieties that are covalently attached to *Wnt* polypeptide when it is produced in the metazoan cell from which it is derived in nature. Native palmitolyation in its turn refers to the covalent attachment of lipid derivatives to a Wnt polypeptide when it is produced in the metazoan cell which is derived in nature (Willert et al, 2003). Accordingly, a human *Wnt* polypeptide produced in a non-metazoan cell is an example of a *Wnt* that is "not associated with native glycosylation or palmitolyation". Sometimes, the *Wnt* polypeptide is unglycosylated or unpalmitolyated (e.g., as a result of being produced recombinantly in a prokaryote).

A "native sequence" polypeptide is a polypeptide that has the same primary amino acid sequence as a polypeptide (e.g., *Wnt* polypeptide) derived from nature. Such native sequence polypeptide is for instance isolated from nature or is produced by recombinant or synthetic means. A native sequence polypeptide has the amino acid sequence of naturally occurring human polypeptide, murine polypeptide, or polypeptide from any other mammalian species.

The term "native sequence *Wnt* polypeptide" includes *Wnt* polypeptides from any animal species (e.g., human, murine, rabbit, cat, cow, sheep, chicken, porcine, equine, etc.) as occurring in nature. The term "native sequence *Wnt* protein" includes the native proteins with or without the initiating N-terminal methionine (Met), and with or without native signal sequence. The native sequence human and murine *Wnt* polypeptides known in the art are from about 348 to about 389 amino acids long in their unprocessed form reflecting variability (particularly at the poorly conserved amino-terminus and several internal sites), contain 21 conserved cysteines, and have the features of a secreted protein (see, e.g., *Wnt* polypeptides as in Gavin et al., supra; Lee et al., supra; Christiansen et al., supra; PCT/US94/14708 [WO 95/17416]). The molecular weight of a *Wnt* polypeptide is about 38-42 kD in a monomeric form. a biologically active *Wnt* variant has an amino acid sequence having at least about 90% amino acid sequence identity with a native sequence *Wnt* polypeptide, preferably at least about 95%, more preferably at least about 99%. Effector functions of native sequence *Wnt* polypeptides preferably include inhibition of differentiation and/or enhancement of proliferation and/or induction of apoptosis.

"Isolated" *Wnt* polypeptide has been purified from a Wnt source or has for instance been prepared by recombinant or synthetic methods and is sufficiently free of other peptides or proteins (1) to show homology for at least 16 and preferably 20 amino acid residues of the N-terminal or of an internal amino acid Wnt polypeptide sequence, or (2) to homogeneity by SDS-PAGE under non-reducing or reducing conditions using Coomassie blue or, preferably, silver stain.

The term "antibody" is used for binding molecules in the broadest sense and amongst other things covers monoclonal antibodies, antibody compositions with poly-epitope specificity, bispecific antibodies, diabodies, and single-chain molecules, as well as antibody fragments (e.g., Fab, F(ab').sub.2, and Fv), so long as they exhibit the desired biological activity.

The term "monoclonal antibody" as used herein refers to an antibody obtained from a population of substantially homogeneous antibodies, i.e., the individual antibodies constituting the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. In addition to their specificity, the use of monoclonal antibodies synthesized by a hybridoma culture is advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention are in one embodiment made by the hybridoma method first described by Kohler et al., Nature 256:495 [1975], and are in another embodiment made by recombinant DNA methods (see, e.g., U.S. Pat. No. 4,816,567 (Cabilly et al.)). In yet another embodiment, the "monoclonal antibodies" are isolated from phage antibody libraries using the techniques in Clackson et al., Nature 352:624-628 [1991] and Marks et al., J. Mol. Biol. 222:581-597 [1991], for example.

The monoclonal antibodies as provided herein also comprise "chimaeric" antibodies (immunoglobulins) in which a portion of the heavy and/or light chain is identical with or homologous to corresponding sequences in antibodies derived from a particular species or identical with or homologous to corresponding sequences in antibodies derived from another species or belonging to another antibody class or subclass, as well as fragments of such antibodies, so long as they exhibit desired biological activity (Cabilly et al., supra; Morrison et al., Proc. Natl. Acad. Sci. USA, 81:6851-6855 [1984]).

The phrase "enhancement of proliferation of a cell" encompasses the step of increasing the extent of growth and/or reproduction of the cell, relative to an untreated cell either *in vitro* or *in vivo*. An increase in cell proliferation in cell culture is for instance detected by counting the number of cells before and after exposure to a molecule of interest. The extent of proliferation is alternatively quantified via microscopic examination of the degree of confluency. Cell proliferation is also quantified using a thymidine or BrdU incorporation assay.

By "controlling differentiation of a cell" is meant the act of increasing the extent of the acquisition or possession of one or more characteristics or functions which differ from that of the original cell (i.e., cell specialization). This is for instance detected by screening for a change in the phenotype of the cell (e.g., identifying morphological changes in the cell and/or surface markers on the cell).

A "lung stem cell or lung progenitor cell" or "primitive lung cell" is a cell, which is able to differentiate to form a more committed or mature lung cell type.

"Mammal" refers to a human or non-human mammal, including, a domestic and farm animal, and a zoo, a sports, or a pet animal, such as a dog, a horse, a cat, a cow, etc. Preferably, the mammal is human.

In one embodiment, the present invention provides the use of a Wnt 3a or Wnt 3a variant having at least 90% amino acid sequence identity with a Wnt 3a. that enhances the canonical Wnt-signaling pathway to levels that prevent or inhibit or modulate or at least retard the differentiation of stem cells from the lung and/or of omni-potent stem cells and enhance their proliferation.

The concentration of Wnt3a or Wnt3a variant having at least 90% amino acid sequence identity with Wnt3a needed to enhance the Wnt-signaling pathway in order to inhibit differentiation and/or to enhance proliferation, is depending on the stem cell type and the relevant factor. As an example, the effective concentration needed for the proliferation of lung cells is disclosed in the examples, but with the method of the invention, a skilled person is taught how to find the effective concentration for other cell types and organ types. For recombinant human Wnt3a as produced by R&D systems (cat nr. 1323-WN) the effective concentration for lung stem cells is between 1 and 2000 ng/ml. Therefore, the invention discloses a method for at least in part inhibiting differentiation of Wng stem cells in a population of mammalian cells comprising providing said stem cell in vitro with a Wnt3a or Wnt3a variant having at least 90% amino acid sequence identity with Wnt3a . Preferably, said upregulating the Wnt signalling pathway is achieved by contacting said cells with a soluble Wnt3a factor.

Nowadays it is possible by differential adhesion and percoll gradient centrifugation techniques to isolate a stem cell population from lung and airway tissue. Long-term culture (for example for more than 4 or 5 days) of these stem cells without loosing the stem cell properties was not possible before this invention.

One embodiment of the present invention provides a method to expand the total number of somatic stem cells present in a population of cells such as for example lung and airway tissue (including the tracheal compartment, the bronchial compartment, the bronchiolar compartment and the alveolar compartment) and of omni-potent stem cells (such as embryonic stem cells) in-vitro, whilst at least in part preserving their original stem cell properties. The invention provides a method for increasing the number of stem cells in a population of mammalian cells compared to a reference population, comprising upregulating a Wnt signaling pathway in said population of cells to a differentiation inhibiting level, wherein said reference population is not provided with said upregulating of a Wnt signaling pathway.

In one embodiment of this invention stem cells are isolated from healthy lung tissue and expanded *in vitro* and used as a source of stem cells that are transplanted directly to a patient in order to generate new lung or airway tissue.

The invention now also enables a skilled person to isolate lung or airway stem cells from a lung sample of the patient's lung or airway tissue and to expand these patient-derived stem cells in sufficient numbers *in vitro* in order to return these stem cells to the patient. Therefore, the invention provides a method for selective differentiation of a stem cell, comprising controlling the level of Wnt pathway activation.

In another example, the disclosure provides genetic modification of the cells of a person with a genetic defect in the lung cells, and it provides a method for isolating, culturing and expanding a patient's own stem cells *in vitro* and to modify them genetically in order to correct the effect of this genetic defect present in the patient's germ line and to return these genetically modified stem cells to the patient. .

In another embodiment, because with the present invention stem cells are cultured for a longer period of time, cultured stem cells from lung and airway tissue are used as a source to generate differentiated lung and airway tissue *in vitro* in order to transplant these to a patient with a lung or airway defect. Therefore in another embodiment, this application describes a method for repopulating cells in a mammal comprising administering to said mammal a therapeutically effective amount of a stem cell or a lung stem cell of the invention. This method is particularly useful for the treatment of people suffering from or susceptible to lung failure, said lung failure for example caused by lung surfactant-deficiency, emphysema, chronic respiratory distress, and/or cancer. The cells are in one example taken out of the body to be treated according to the present invention, or one chooses to treat the cells in the body. Therefore, the present invention discloses a method for at least in part inhibiting differentiation of stem cells, and/or increasing the numbers of stem cells, and/or enhancing the apoptosis of mesenchymal cells, and/or differentiating the stem cells in the mammalian body, preferably in a human body, comprising manipulating the Wnt pathway. In another embodiment the invention provides a method to prevent or retard the differentiation of omnipotent stem cells. Such omni-potent stem cells have the ability to differentiate into all the possible cell-types and cell-lineages that are present in the full-grown organism. Omni-potent stem cells are preferably isolated from the inner-cell mass of early embryos at the blasto-cyst stage. The invention is also valid for omni-potent stem cells that can be isolated from early embryos at other developmental stages than the blastocyst stage. The invention is also suitable for omni-potent stem cells, that are isolated from the genital ridge, the so called primordial germ cells (PGCs), of early embryos that already underwent the formation of the three germ layers: ectoderm, endoderm and mesoderm during gastrulation. The invention is also suitable to omni-potent stem cells that are obtained by the transfer of nuclei from differentiated somatic cells (preferably from a patient) to one of the omni-potent stem cells described in this patent.

One of the applications in which omni-potent stem cells are used is the generation of transgenic animals by transfecting an omni-potent cell with a DNA-construct in such a way that the original DNA content of that cell is changed. Such a DNA construct is maintained in the cell by an interchromosomal integration (nuclear or mitochondrial) or as an episomal (extra-nuclear) DNA molecule. Maintenance of the omni-potent cells is facilitated by the addition of leukocyte inhibitory factor or other growth factors, preferably added in a purified form or produced by a cell-line. The present invention provides an alternative to these growth factors as addition of a Wnt pathway elevating factor at an effective concentration helps to maintain the omni-potent characteristics of the stem cells.

Another example of this disclosure provides a method for the generation and isolation of omni-potent stem cells from the embryo. Omnipotent stem cells are isolated from the embryo at different developmental stages. The difficulty in this process is the loss of the omni-potent properties of the stem cells due to differentiation into various cell-types. This disclosure at least prevents or retards the differentiation of these omni-potent stem cells thereby facilitating the isolation of omni-potent stem cell lines from embryos whilst maintaining their omni-potent properties. Isolation of omni potent stem cells from early embryos was before this invention very difficult, if possible at all, in many species including humans. This disclosure now allows for isolation of omni-potent stem cells from various mammalian species including humans with a much higher frequency and efficiency than previously was possible.

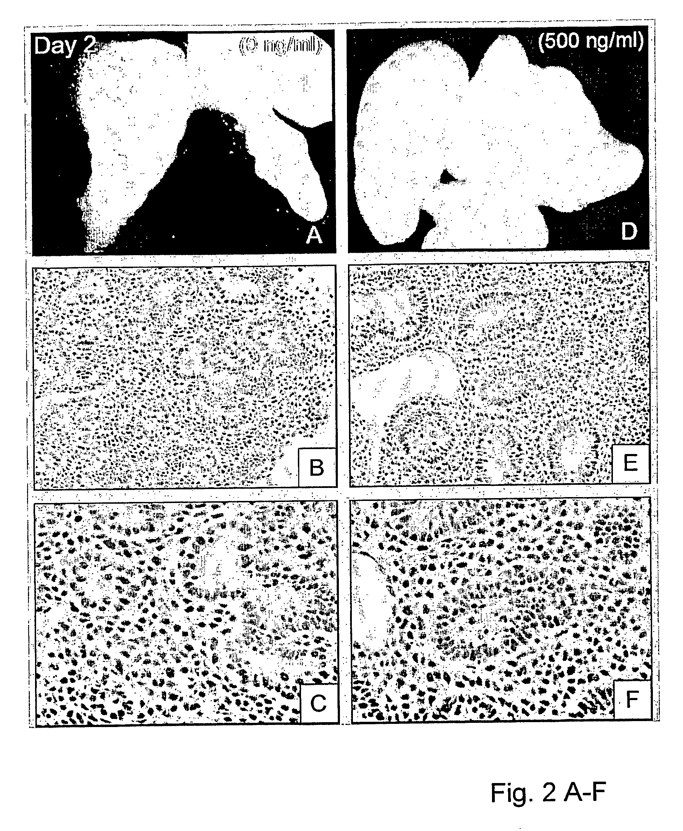

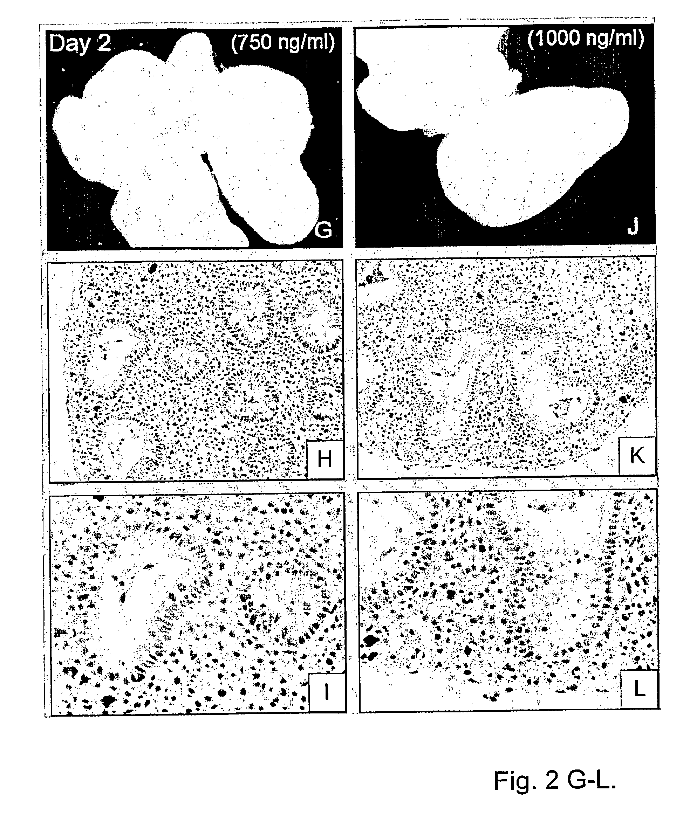

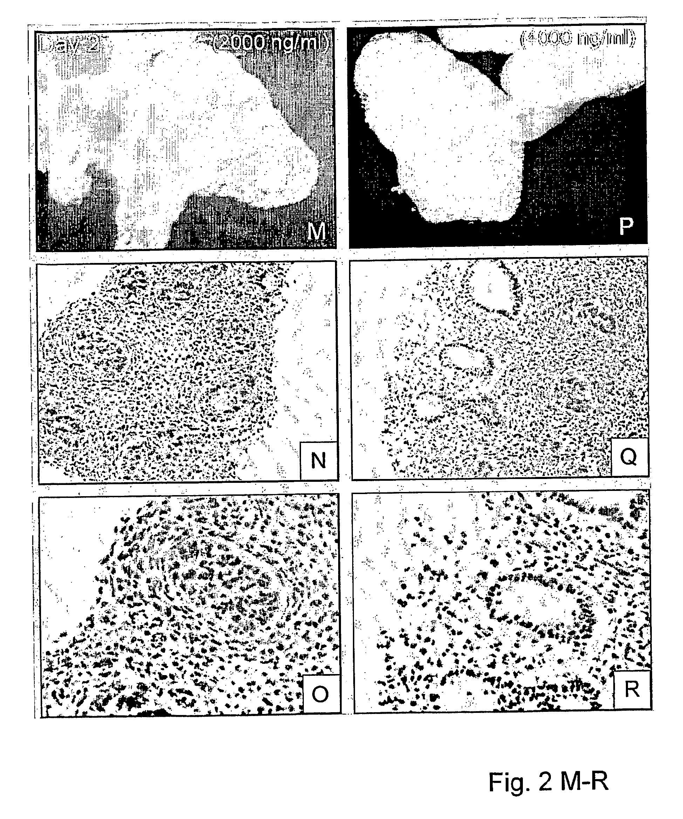

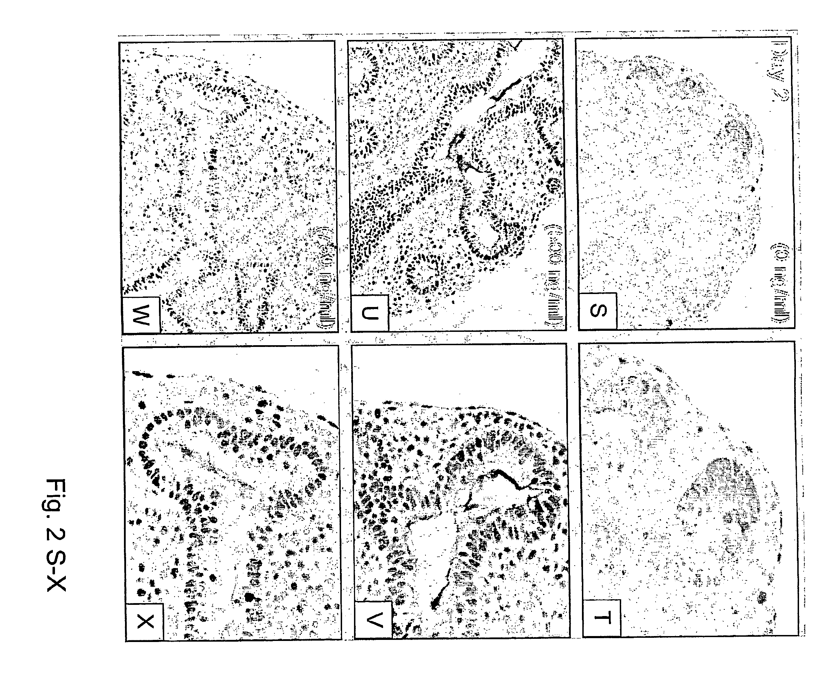

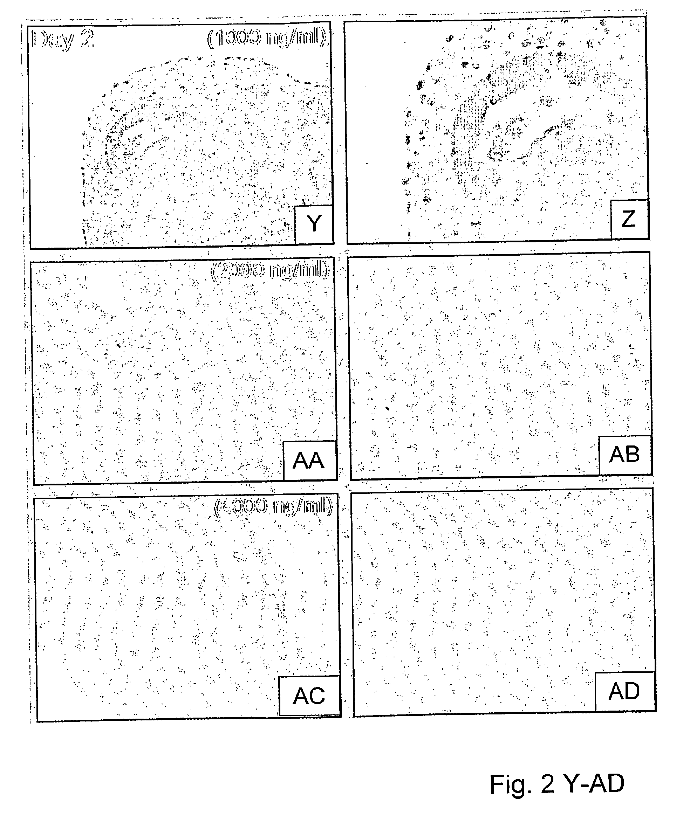

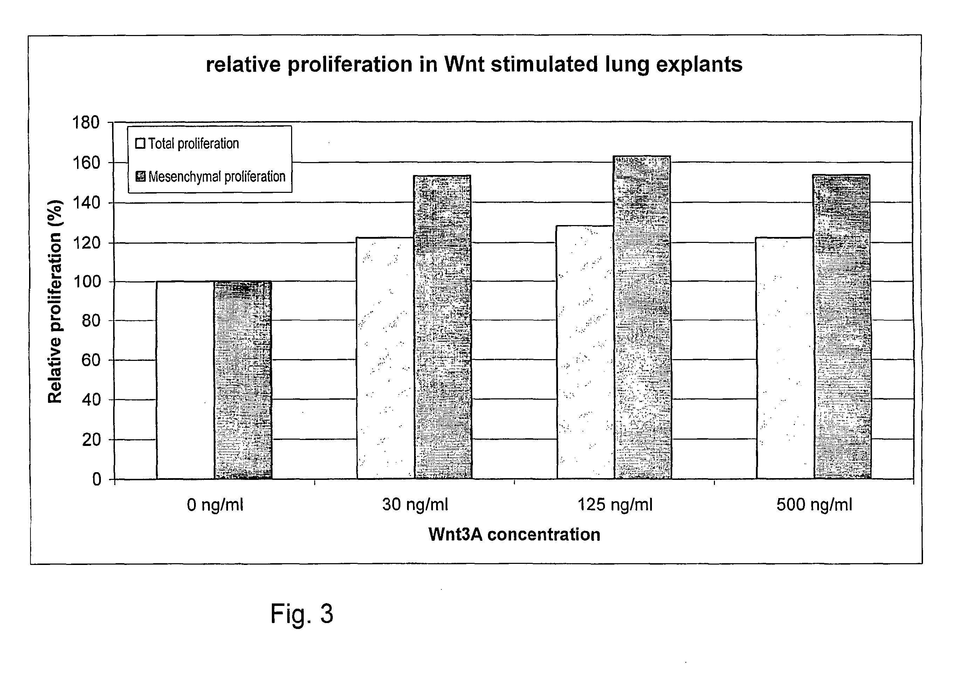

In another embodiment, the invention provides the insight that the induction of apoptosis by activation of the Wnt pathway is possible in every cell type present in the lung, i.e. terminally differentiated cells as well as stem cells. The concentration of a Wnt 3a or Wnt 3a variant having at least 90% amino acid sequence identity with Wnt 3a needed to induce apoptosis in a specific cell-type is dependent on the factor itself and on the cell-type. This concentration is for cell-types present in the lung disclosed in example 1, figure 2 and 3, The level of Wnt 3a polypeptide needed for apoptosis of mesenchymal cells is close to the level needed for inhibition of the differentiation and the enhancement of stem cell proliferation. Therefore, the present application discloses a method for inducing apoptosis in mesenchymal cell in a population of mammalian cells comprising providing said mesenchymal cell in vitro with a Wnt 3a or Wnt 3a variant having at least 90% amino acid sequence identity with Wnt 3a. . Preferably, said population also comprises a stem cell.

Apoptosis of mesenchymal cells prevents overgrowth of the somatic stem cells during their culture *in vitro* thereby facilitating the purification and selective culture of lung stem cells *in vitro.*

Differentiation of omni- or pluri-potent stem cells is dependent on the strength of the Wnt signaling pathway in these cells. Stem cells in which the Wnt signaling pathway is activated are limited in their differentiation capacity by the action of the Wnt signal. A stem cell in which the possible differentiating capacity or amplitude of the Wnt signaling pathway is maximal can differentiate to all its derivatives. A stem cells in which the amplitude of the Wnt-signaling pathway is limited to a certain window is limited in its differentiation capacity and is restricted to differentiate to certain cell-types.

This invention provides a method to direct the differentiation of omnipotent and pluripotent stem cells to a limited number of desired cell types such as for example bronchial epithelial cells or tracheal epithelial cells or epithelial alveolar type I or-II cells by restricting the Wnt signal strength to a certain window. As already described before, the present disclosure teaches how to direct differentiation in an organ or organ culture in vitro. Therefore, the present disclosure provides a method wherein said population of cells is arranged in a three dimensional structure. This disclosure further provides a soluble compound capable of influencing the propagation or the selected differentiation of a stem cell. Said soluble compound for example comprises a soluble factor capable of upregulating a Wnt signaling pathway in said cells, such as for example an isolated Wnt factor or an antibody or a monoclonal antibody capable of specifically binding and acting like a Wnt factor.

Therefore, the present inventions discloses a method of the invention wherein said Wnt 3a comprises a soluble factor capable of upregulating a Wnt signaling pathway in said cells, and or a soluble isolated Wnt 3a-factor, Therefore, the present invention also discloses a method of the invention, wherein said factor comprises Wnt-3a or Wnt 3a variant having at least 90% amino acid sequence identity with Wnt 3a

The present invention discloses a range of concentrations at which a Wnt3a or Wnt 3a variant having at least 90% amino acid sequence identity with Wnt 3a is effective in inhibiting at least partial differentiation of stem cells in a population. Therefore, the present invention discloses a method for inhibiting differentiation of a lung stem cell, comprising providing said stem cell in vitro with a Wnt3a or Wnt 3a variant having at least 90% amino acid sequence identity with Wnt 3a .

The present invention also provides the insight that the stem cells are stimulated to proliferate by the presence of said soluble Wnt factor. Therefore, the invention also teaches a method for inducing a stem cell, preferably a lung stem cell, to proliferate by inhibiting differentiation of said stem cell, comprising providing said stem cell in vitro with a Wnt-pathway upregulating substance. In-one embodiment the invention provides a method of the invention, wherein said Wnt-pathway upregulating substance comprises a soluble Vnt3a factor or a functional part, derivative or analogue thereof. The invention furthermore disclose in the examples that Wnt3a or Wnt 3a variant having at least 90% amino acid sequence identity with Wnt 3a already upregulates a Wnt pathway from 1 ng/ml tissue culture fluid, and is still upregulating at 2000 ng/ml. Therefore, the invention provides a method of the invention, wherein said stem cell is provided with Wnt3a or Wnt 3a variant having at least 90% amino acid sequence identity with Wnt 3a in an amount of at least 1 ng and at most 2000 ng per ml tissue culture fluid. It is in the examples that the effects of Wnt3a on a Wnt pathway vary with the concentration of Wnt3a. Therefore, in another preferred embodiment, the invention teaches said above described method wherein the amount of Wnt3a or Wnt 3a variant having at least 90% amino acid sequence identity with Wnt 3a is from at least 10 to at most 1500 ng per ml tissue culture fluid. Said amount of Wnt3a is more preferable from at least 20 to at most 1000 ng per ml tissue culture fluid, most preferably from at least 30 to at most 600 ng per ml tissue culture fluid.

In another embodiment, the present invention provides the insight that at a certain level of Wnt upregulation apoptosis in mesenchymal cells occurs while epithelial cells are not affected. This characteristic of Wnt upregulation is used to selectively remove cells from a population of cells by choosing the level of Wnt upregulation such that apoptosis in mesenchymal cells is induced while epithelial cells are not affected. Therefore, the present invention provides a method for enriching a population of lung stem cells in a population of mesenchymal cells comprising said lung stem cells, by inducing apoptosis in mesenchymal cells by providing said mesenchymal cells with a Wnt 3a or Wnt 3a variant having at least 90% amino acid sequence identity with Wnt 3a .

Because the level of upregulation of the Wnt pathway is dependent on the amount of Wnt3a administered to the cells, the invention also provides a method of the invention, wherein said mesenchymal stem cell is provided with Wnt3a or Wnt 3a variant having at least 90% amino acid sequence identity with Wnt 3a in an amount of at least 60 ng and at most 1500 ng per ml tissue culture fluid, such that mesenchymal cells are becoming apoptotic.

The examples disclose that the level of apoptosis is changed by changing the level of Wnt pathway upregulation, therefore, in a more preferred embodiment of the invention, a method of the invention is disclosed, wherein said mesenchymal stem cell is provided with Wnt3a or Wnt 3a variant having at least 90% amino acid sequence identity with Wnt 3a, in an amount of at least 100 ng and at most 1000 ng per ml tissue culture fluid.

In another embodiment of the invention, the present invention also provides selective differentiation of lung stem cells that is dependent on the upregulation of the Wnt- pathway. Therefore, the present invention also provides a method for providing a plurality of lung stem cells from a population of mesenchymal cells and stem cells, comprising providing said mesenchymal cells with an apoptosis inducing amount of a Wnt3a or Wnt 3a variant having at least 90% amino acid sequence identity with Wnt 3a .

Of course the above described method is very suitable for lung stem cells, therefore, a preferred embodiment of the invention provides a method of the invention, wherein said stem cells are lung stem cells.

The present invention provides the insight that Wnt3a is very suitable for upregulating the Wnt-pathway.

An amount of Wnt3a between 50 ng and 1600 ng is preferred in order to achieve optimal results. Therefore, the present invention provides a method of the invention, wherein said soluble Wnt3a or Wnt 3a variant having at least 90% amino acid sequence identity with Wnt 3a, is provided in an amount of at least 50 ng and at most 1600 ng per ml tissue culture fluid.

In a more preferred embodiment, the invention provides a method of the invention, wherein differentiation of a distal lung cell type is at least partly inhibited by administering 10- 250 ng Wnt3a or Wnt 3a variant having at least 90% amino acid sequence identity with Wnt 3a ml tissue culture fluid.

Said distal lung cell type is preferably an alveolar type cell, more preferable a type I or type II alveolar type cell. Therefore, the present invention provides a method of the invention, wherein said distal lung cell type is a type I or type II alveolar cell.

Stem cells are capable of differentiating into various types of cells originating from a stem cell present in a population of lung stem cells. Upregulation of the Wnt pathway influences that differentiation as is disclosed in the examples. Therefore, the present invention also provides a method of the invention wherein proliferation and differentiation of an upper airway cell type is selectively increased by administering 10-750 ng Wnt3a or Wnt3a variant having at least 90% amino acid sequence identity with Wnt 3a /ml tissue culture fluid. In a preferred embodiment, said upper airway cell type is a tracheal or bronchial epithelial cell. Therefore, the present invention also provides a of the invention, wherein said upper airway cell type is a tracheal and/or bronchial epithelial cell.

Wnt3a is a soluble compound that up regulates the Wnt pathway in cells. Such a compound is preferably included in a cell culture medium.

### EXAMPLES

### Example 1

### Generation of new lung tissue ex vivo

### Preservation and potential growth of alveolar tissue in a murine lung by in culture treatment with recombinant Wnt3a polypeptide.

To provide proof of evidence, an in vitro explant lung culture (obtained from a mouse) is used. Generation of new alveolar tissue in patients is stimulated by activation or re-activation of alveolar bud formation. Growth and branching of alveolar buds, followed by transformation into alveolar ducts, sacs and pouches, results in the establishment of pulmonary acini.

This is a general differentiation principle in both the foetal and the adult mammal.

As a proof of evidence, it is therefore shown that treatment with selected molecules, involved in a Wnt-pathway, inhibits the differentiation process of alveolar differentiation. Inhibition of this differentiation process is achieved by applying a purified Wnt3a polypeptide to an in vitro lung explant culture of 13-day-old (E13) mouse embryos.

### Material& Methods

### In vitro lung explant cultures

The (foetal) murine lung explant culture was generated according to our standard protocol. Briefly, complete lungs or individually dissected lung lobes were isolated from foetal mice and cultured for 1 to 5 days in a hanging drop culture system. The Wnt3a polypeptide was administered to the culture medium in different concentrations, ranging from 0-4000 ng/ml. The effect of this treatment was monitored by stereomicroscopy. The lung explant cultures were subsequently fixed for 30 minutes in 10% paraformaldehyde (PFA), 30 minutes in 2% PFA and 30 minute sin 4% PFA. After fixation the lung explants were dehydrated through an increasing series of 2-isopropanol and subsequently embedded in paraffin. For the histological analysis 4um thick sections were cut and stained with Hematoxilin and Eosin using standard techniques.

For the immunohistochemical analysis comparable sections were used and treated with and anti-PCNA monoclonal antibody followed by a goat antimouse horse reddish peroxidase conjugate. Detection of the PCNA epitopes was carried out by treating the sections with di-aminobenzidine (DAB) for 15'. TopFlash/FopFlash reporter system:
Primary mouse fibroblasts isolated from E12.5 lung explants, the human epithelial lung carcinoma cell line A549 cells (ATCC *CCL-185*), *the human epithelial lung adenocarcinoma cell line Calu-3* (*ATCC_HTB-55*) and the normal human foetal lung fibroblast cell line HFL-1 cells (ATCC_153) were cultured in 96-well plates and transfected with the TopFlash and FopFlash reporter constructs using lipofectamine 2000 according to the manufacturer instructions. As an internal control for transfection efficiency a constitutive renilla luciferase reporter (promega) was cotransfected. The cells were transfected with the above mentioned reporter constructs and simultaneously treated with different concentrations of Wnt3A for 20 hours, after which the cells were lysed and luciferase expression was measured using a Berthold luminometer for 2 seconds (Korinek, V., et al., Mol. Cell. Biol. 18: 1248-1256, 1998). The Lipofectamine2000 transfection reagent was purchased from Invitrogen life technologies. The wild type beta-catenin/TCF reporter plasmid (TOPFLASH) and mutated reporter plasmid (FOPFLASH) were obtained from Upstate biotechnology (NY, USA).
Quantification of cell proliferation using phospo Histon H3 immunohistochemistry. The number of cells undergoing mitosis were measured using immunohistochemical detection of phosphorylated histon H3 which is present only on condensed chromosomes during mitosis. Per condition at least four different lung explants were treated and in total 20 sections per condition were counted for phosphor histon H3 positive cells by two different investigators. The primary antibody against phopho histon H3 (Ser10, clone 6G3) was purchased from Cell Signaling technology and used according the recommendations of the supplier:
The induction of apoptosis was detected using the *"In Situ* Cell Death Detection Kit, of Roche (Cat. No. 11 684 817 001) according the manufacturer instructions.
As a source for Wnt3a was used, recombinant mouse Wnt-3a from R&D systems (catalog Nr: 1324-WN, R&D systems, Inc. Minneapolis USA). As culture medium was used DMEM/HAM's F12 (1:1) from Biochrom AG, catalog nr. FG 4815.

The antibody against PCNA was purchased from Zymed Laboratories Inc., (cat nr.18-0110). The secondary conjugate used was from Jackson Laboratories, Inc (catalog nr 115-056-062).
The primary antibodies used for the immunohistochemical detection was for beta-catenin; anti beta-catenin IgG, mAb, clone 14 from BD-Transduction Laboratories, for tubulin-beta mAb clone TBN05 (Tub2.1) from Neomarkers, for BrdU; anti-Bromodeoxyuridine (BrdU), mAb clone ZBU30 from Zymed laboratories inc.

### The effect of 500 ng/ml to 4000 ng/ml Wnt3A on the growth of foetal lung explants.

### Results:

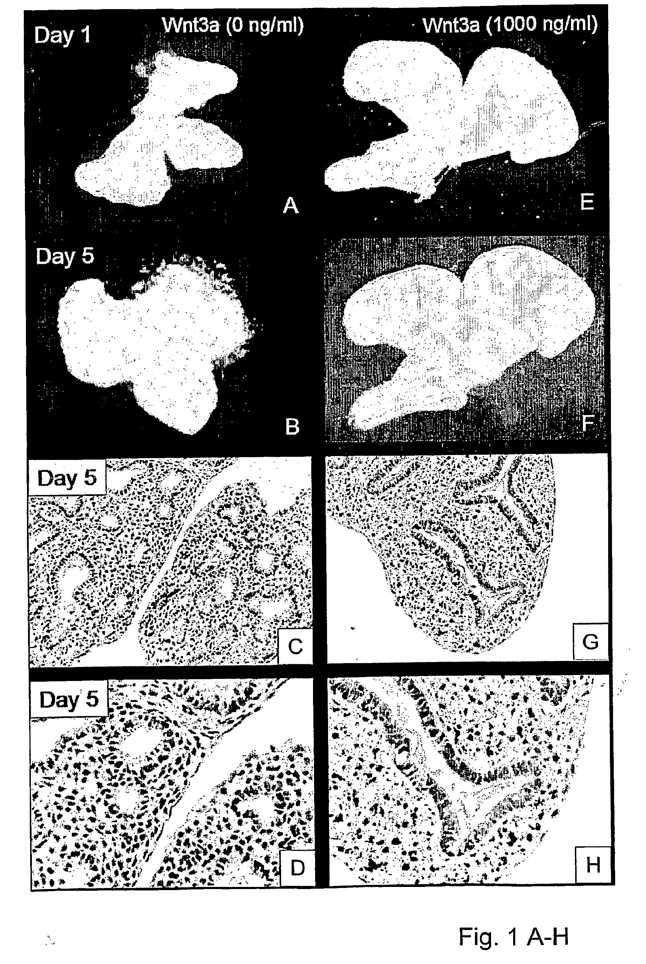

Treatment of the lung cultures of embryonic day 13 (E13) lung explants with 1000 ng/ml Wnt3a polypeptide resulted in a total block of lung differentiation after 16 hours (Fig. 1E). Even after five days culture under these conditions no significant differentiation and alveolar development could be observed by stereomicroscopy (Fig. 1F).

Histological analysis of these five day old cultures revealed that the differentiation of the primordial lung epithelium was arrested at a stage comparable to that of the start of the culture (E13). Furthermore, most of the mesenchymal cells surrounding the epithelium displayed picnotic nuclei, indicating that these cells had undergone apoptosis. This was in sharp contrast with the epithelium that contained no apoptotic cells (Fig. 1H).

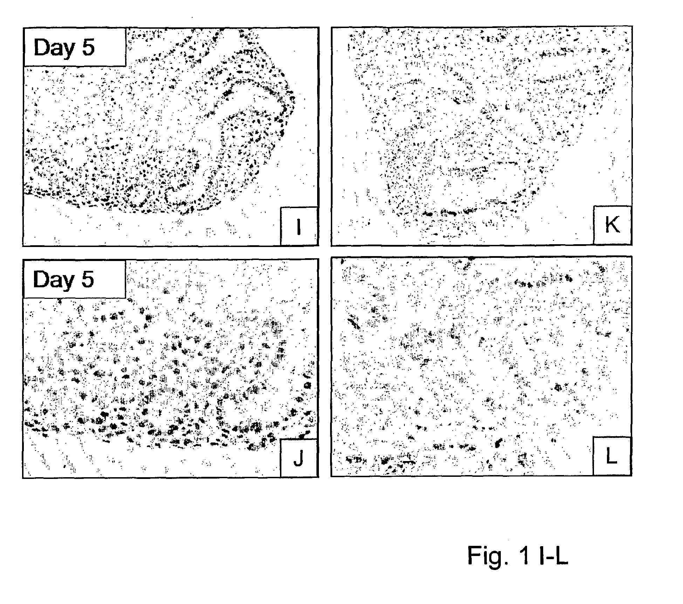

To analyze the viability of the epithelium and the surrounding mesenchym, the sections were stained for the proliferation marker "Proliferating Cell Nuclear Antigen" (PCNA, Fig 1I-J). This immunohistochemical staining showed that the epithelium was still proliferating whereas most of the mesenchymal cells were inactive or dead (Fig. 1K+L). This in contrast to the untreated lung explant cultures were both the epithelium and the mesenchym were strongly proliferating (Fig 1I+J).

Above results indicate that the Wnt3a polypeptide has a strong inhibitory effect on the development and differentiation of the foetal lung.

In order to determine the concentration range in which the Wnt3a polypeptide is effective total lung explants were cultured in the presence of Wnt3a in a range of 0 ng/ml, 500 ng/ml, 750 ng/ml, 1000 ng/ml, 2000, ng/ml and 4000 ng/ml Fig. 2A-R).

Stereomicroscopic analysis showed already after one day a deleterious effect on lung development with the Wnt3a concentrations, 2000 ng/ml and 4000 ng/ml (Fig 2M+P).

After 2 days of culture, these lung explants were fixed and embedded in paraffin for immunohistological analysis.

The histological analysis of H/E stained sections showed that the mesenchymal cell population undergoes a rapid induction of apoptosis within the range 1000-4000 ng/ml Wnt3a. Apoptosis is also induced in the epithelium at a concentration of 2000-4000 ng/ml but is not detectable at a concentration of 1000 ng/ml. The mesenchym however, becomes at a concentration higher than 750 ng/ml clearly apoptotic indicating that the induction threshold for apoptosis is different for the epithelium and the mesenchym. At the concentration of 750 ng/ml apoptosis in the mesenchym is clearly detectable but not yet abundant, whereas the epithelium showed no sign of apoptosis whatsoever Fig. 2H+I).

Immunohistochemical staining of PCNA showed in addition a Wnt3a dependent proliferation upon the lung explant cultures. Without addition of Wnt3a the periphery of the lung containing the growing lung buds show a stronger staining for PCNA than the more central located bronchial epithelium, indicating that the periphery of the lung is stronger proliferating than the central part (Fig 2S+T). Addition of 500 ng/ml Wnt3a to the medium, however, shows a marked overall increase in the intensity and frequency of PCNA staining in the centrally located bronchial epithelium indicating that proliferation is enhanced by Wnt3a at a dosage of 500 ng/ml Wnt3a (Fig 2U+V). A further increase of the Wnt3a concentration to 750 ng/ml (Fig. 2W+X) does not lead to a further increase the intensity and frequency of the PCNA staining but instead is comparable to the control situation (Fig 2S+T). Addition of 1000 ng/ml Wnt3a reduces the PCNA staining below the control situation (Fig 2Y+Z), whereas at 2000 and 4000 ng/ml Wnt3a (Fig 2AA+AD), PCNA staining was completely negative indicating that proliferation was completely blocked, probably due to the apoptosis induced in the mesenchym and epithelium (Fig. 2).
To further determine the short term effect of different Wnt3A concentrations upon the induction of proliferation, lung explants (E12.5) were cultured for 1 day with 0, 30, 125 and 500 ng/ml Wnt3a.
The lung explants were processed for immunohistochemical analysis and stained for Histon H3 phosphorylation, which can be used as a quantifiable cell proliferation marker as it is present in a fixed amount on condensed chromosomes only during active cell division (material & methods). The results of this quantification show that Wnt3A has a significant stimulatory effect upon proliferation. A more than 20 % increase overall proliferation was observed in the 30, 125 and 500 ng/ml treated Wnt3A samples. However most of the increase in proliferation could be accounted to the mesenchym where an increase in 40 to 60 % of cell undergoing apoptosis was observed. Little difference was found between the different Wnt3A concentrations indicating that the maximum stimulatory effect of Wnt3A was already reached with 30 ng/ml Wnt3a (Fig. 3)_

### The effect of 4 ng/ml to 500 ng/ml Wnt3A on foetal lung growth over four days.

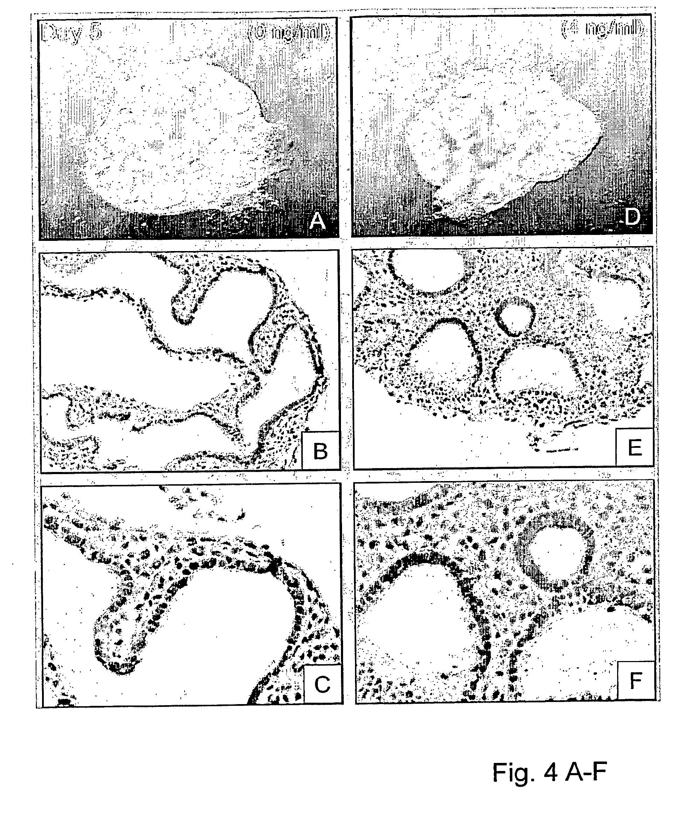

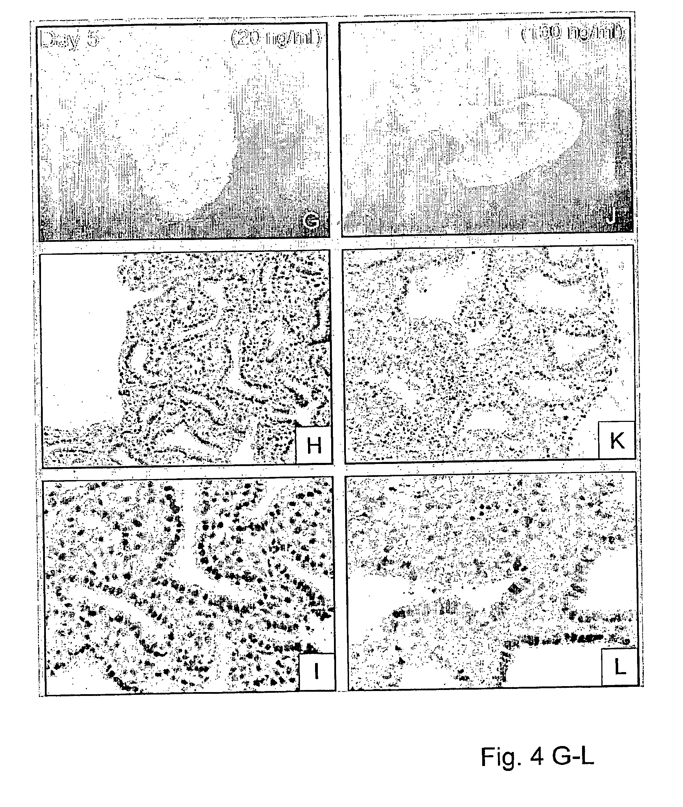

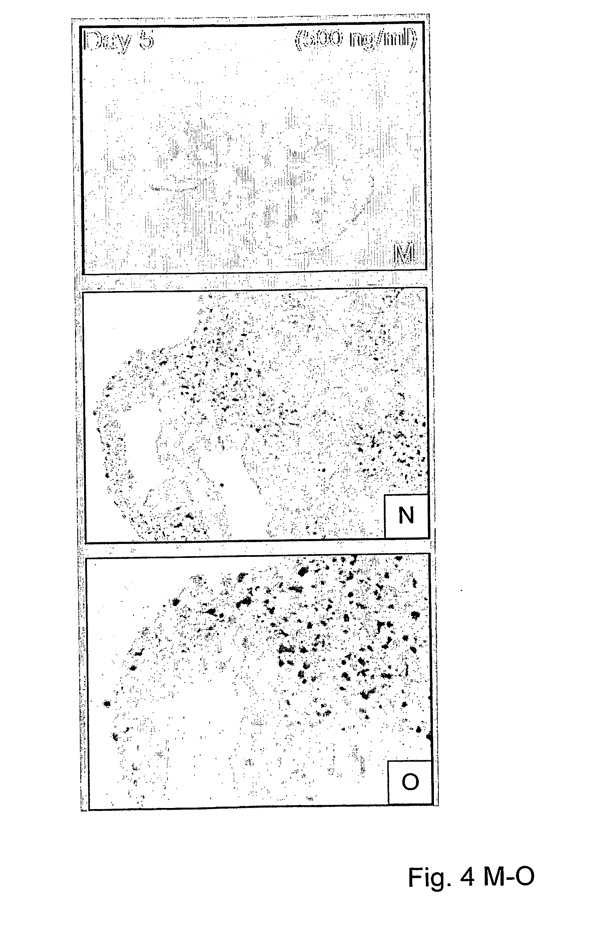

To more exactly determine the concentration of Wnt3a that is effective upon lung development, differentiation and apoptosis, foetal lung explants were cultured for a longer period (4 days) in the presence of lower concentrations of Wnt3a, i.e.; 0 ng/ml, 4ng/ml, 20ng/ml, 100ng/ml and 500ng/ml (Fig 4A-O).

None of these concentrations was effective in completely blocking the differentiation of foetal lung development, although differentiation at a concentration of 500 ng/ml (Fig 4N+O) was delayed as was displayed by the primordial morphology of the epithelium compared to the control situation (Fig 4B+C). Between 100-500 ng/ml the mesenchym showed an increasing level of apoptosis, whereas in the epithelium no sign of apoptosis could be detected (Fig. 4K+L and 4N+O).

Between 4 and 20 ng/ml no clear effects were visible at the level of apoptosis and differentiation compared to the control situation (Fig. 4B+C, 4E+F and 4H+I)).

### Analysis of, apoptosis by the TUNEL assay and active proliferation by BrdU incorporation on lung explants treated with 4ng/ml to 1000 ng/ml Wnt3A during 4 days.

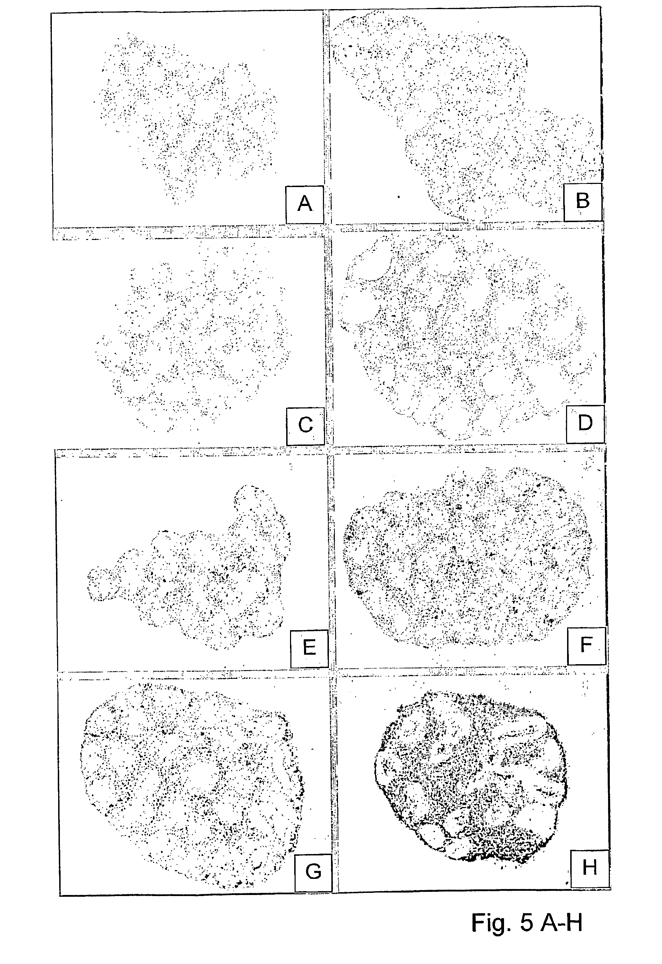

The above described experiments show that the visible biological effect of Wnt3A upon proliferation, apoptosis, and differentiation lies between a concentration range of 1 and 2000 ng/ml. A concentration of 2000 ng/ml is not preferred since it affects the viability of all cells, and thus lacks specificity. A concentration below 1 ng/ml does not show clear morphological changes although a concentration of 30 ng/ml markedly increases proliferation after 1 day. Drastic effects were observed at the level of apoptosis, proliferation and differentiation. To further analyze the effect of Wnt3a upon apoptosis and proliferation, lung explants were cultured for 4 days in the presence of 0, 4, 20, 100, 250, 500, 750 and 1000 ng/ml. In order to identify whether after Wnt3A treatment cells are viable and active, proliferating BrdU was added to the culture medium which is incorporated in newly synthesized DNA of active proliferating cells, two hours before the fixation of the lung explants. Histological examination at day 4 showed, as before, that the process of differentiation was strongly retarded and revealed many picnotic cells at Wnt3a concentrations above 100 ng/ml. To better investigate this effect, histological sections were analyzed with the TUNEL assay, which detects fragmented DNA present in apoptotic cells (material & methods). A low background of TUNEL positive cells was detected in the sham treated lung explants, however a dramatic increased in TUNEL positive cells was observed at concentrations above 100 ng/ml Wnt3a which is proportional to the number of picnotic cells found in these samples (Figure 5). At 1000 ng/ml almost 100% of the mesenchymal cells were picnotic and were also TUNEL positive indicating that they had undergone apoptosis (Figure 4H). In contrast, almost none of the epithelial cells were TUNEL positive, or contained picnotic nuclei, indicating that these cells are much less sensitive for apoptosis compared to the mesenchymal cells (Figure 5).

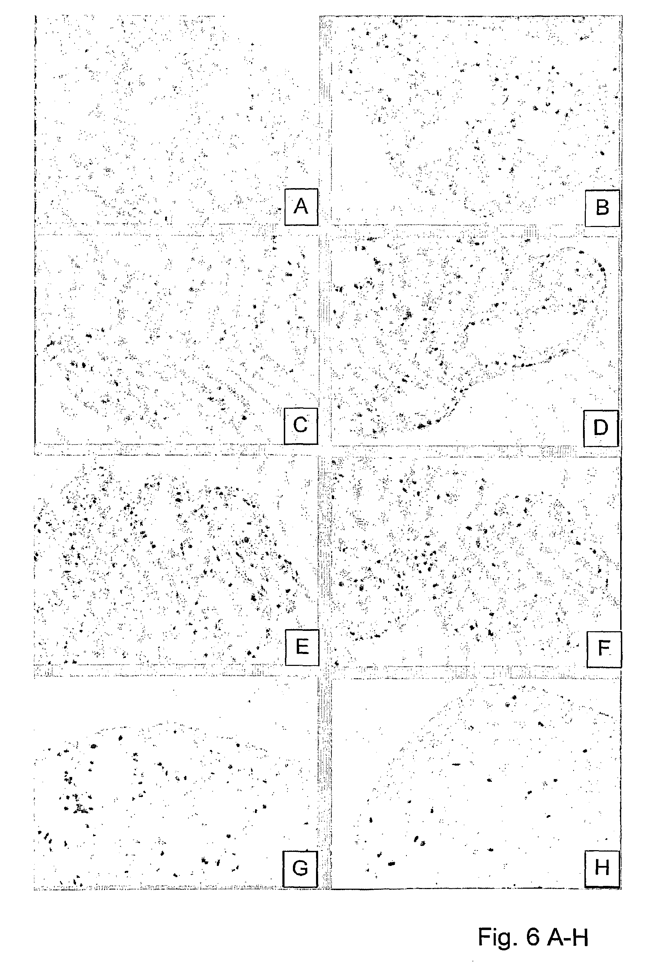

In order to identify whether the epithelial cells were still actively proliferating after four days of Wnt3a treatment, the histological sections were immunostained for incorporated BrdU (Figure 6). At concentrations of 500ng/ml and lower, both the mesenchym and epithelium contained many BrdU positive, thus active proliferating cells. At 1000 ng/ml almost all BrdU positive cells were present in the epithelium and only a very few were present in the mesenchym, indicating that despite the massive induction of apoptosis in the mesenchym, the epithelium is viable and proliferating. At 750 ng/ml Wnt3a an intermediate effect was observed between the 500 and 1000 ng/ml Wt3A treated lung explants (Figure 6)

### Long term treatment of foetal lung explant cultures with Wnt3a induces a dosage dependent shift of distal (alveolar) lung differentiation to proximal (bronchial/tracheal) lung differentiation.

To further determine the effect of Wnt3A upon the development and differentiation of the lung over time, foetal lung explants of isolated at E12.5 were cultured with 0, 10, 50, 250, and 500 ng/ml Wnt3A for 8 days. Two hours before the fixation of the lung explants, BrdU was added to the culture medium which is incorporated in newly synthesized DNA of active proliferating cells. After the different time points the lung explants were processed for histological analysis and immuno staining of marker genes.

### Eight day culture without Wnt3A:

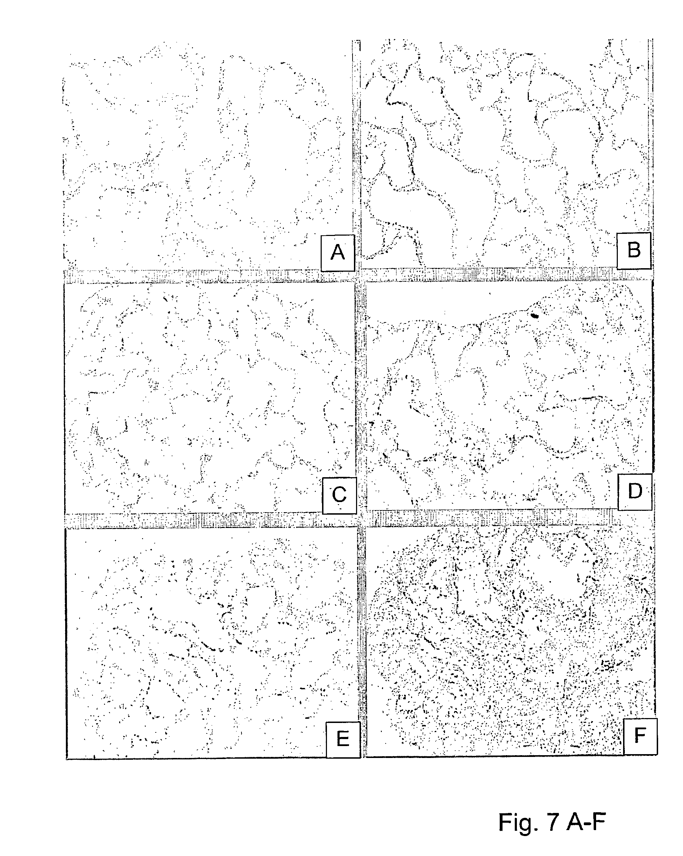

The histological analysis showed that the long term lung development in the hanging drop culture system without Wnt3A addition to the medium, is comparable to in-vivo lung development. The lung explants without Wnt3A could be cultured for eight days without the sign of necrosis or apoptosis. Contraction of the bronchial epithelium is visible after one to two days of culture indicating that smooth muscle cells develop along the conducting airways. This was confirmed with immuno histochemical staining for smooth-muscle actin (data not shown). Several differentiated cell types such as cubical type-II cells, secretory Clara cells, cartilage and the alveolar type-I cells could be clearly distinguished after eight days (Figure 7A). This is not much delayed from the in-vivo situation were alveolar type-I cells differentiate between E17.5 and E19.5, thus corresponding to day 5 and 7 of the in-vitro culture.

### Eight day culture with 10 to 250 nglml Wnt3A.

Treatment of the E12.5 day lung explants showed a clear change in morphology, which became more and more apparent over time, indicating that a Wnt3A concentration as little as 10 ng/ml has profound effects on lung development. Histological analysis of these explants after eight days showed a decreasing alveolarization of the distal epithelium. The normal flattening of the primordial epithelium to the type-I cell which occurs during normal distal lung differentiation is inhibited with increasing Wnt3A concentration and at 250 ng/ml almost completely suppressed (Figure 7B- 7E). Distal lung development is thus inhibited by Wnt3A treatment in a concentration range of, at least, 10ng/ml to 250ng/ml Wnt3A.

### Eight day culture with 500 ng/ml Wnt3A.

Addition of Wnt3A has a striking effect on the final differentiation of E12.5 lung explants. Previous cultures had already shown the short term inhibitory effect upon branching of 500 ng/ml Wnt3A after 1 day. After eight days treatment with 500 ng/ml Wnt3A, branching was still severely suppressed and resembled in morphology the original isolated E12.5 lung explants. However, histological analysis and immunostaining with an anti-tubulin IV antibody which marks ciliated epithelium, showed that these explants consist completely of proximal structures comprising; bronchial epithelium composed of ciliated cells and secretory cells; cartilage and mesenchym. No type-I or type-II cells could be observed indicating that the differentiation of distal epithelium comprising the alveolar and bronchiolar compartments was completely inhibited by the treatment with 500 ng/ml Wnt3A (Figure 7F). The administration of concentrations higher than 250 ng/ml Wnt3A thus restricts lung differentiation to cell types found in the upper airways and is thus suitable for selectively growing proximal lung cell types from primordial lung tissue, an application which is very useful to generate tissue to treat diseases or injuries of the upper airways.

### Wnt3a induces the accumulation of nuclear beta-catenin in lung mesenchymal cells.

In order to determine whether Wnt3A actually elevates the Wnt-signaling pathway in these explants, histological sections were stained for beta-catenin, the intracellular factor which together with members of the high mobility group (HMG) of transcription factors such as TCF1 and LEF, transduces the Wnt-signal to the nucleus.

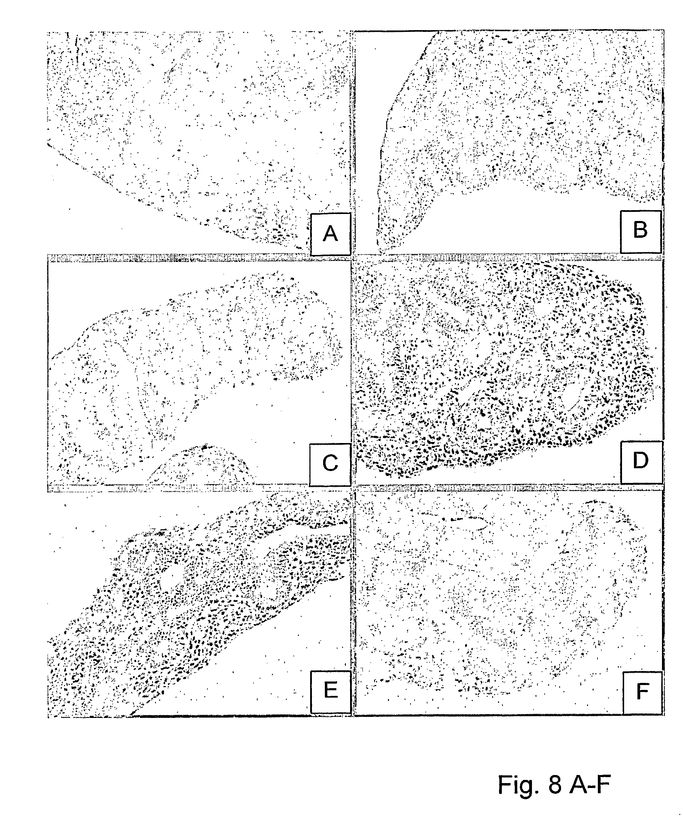

The results showed that there is a Wnt3A dosage dependent increase in nuclear beta-catenin within the first two days which was especially prominent in the mesenchymal cells surrounding the growing and branching lung buds in the distal lung compartment. This effect was maximal in the 250 and 500 ng/ml Wnt3A treated lungs (Fig. 8D + 8E), and had disappeared in the four and eight day cultures (data not shown).

### Wnt3a induces a dosage dependent activation of Wnt-reporter expression in foetal lung tissue.

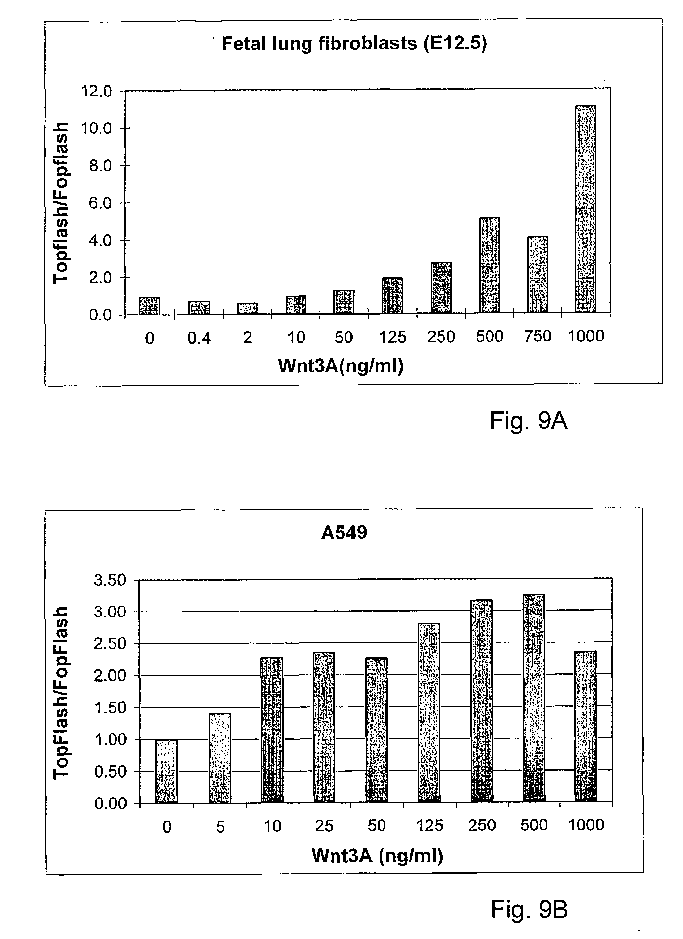

Above results indicate that foetal lung tissue is capable of transducing the Wnt-signal by addition of Wnt3A to the culture. To further prove that Wnt3A is capable of inducing the wnt-signaling pathway in lung tissue, we conducted the TopFlash/FopFlash reporter assay on dissociated lung explants isolated from E12.5 embryos. The dissociated lungs were co-transfected with either the TopFlash reporter construct which is activated by nuclear beta-catenin transcription complexes. This was compared to the activation of a FopFlash reporter construct which is mutated in the beta-catenin/TCF binding sites and measures only the background expression from the minimal promoter (Korinek, V., et al., Mol. Cell. Biol. 18: 1248-1256, 1998). The ratio TopFlash expression versus FopFlash expression is a measure for the induction of the Wnt-signaling pathway by Wnt3A. The transfected cells were treated with different concentrations of Wnt3A ranging between 0,4 to 500 ng/ml Wnt. A clear Wnt3A dosage dependent response was observed indicating that the foetal lung cells have all the necessary components to receive and transduce the wnt-signal transduction pathway (Figure 9A).

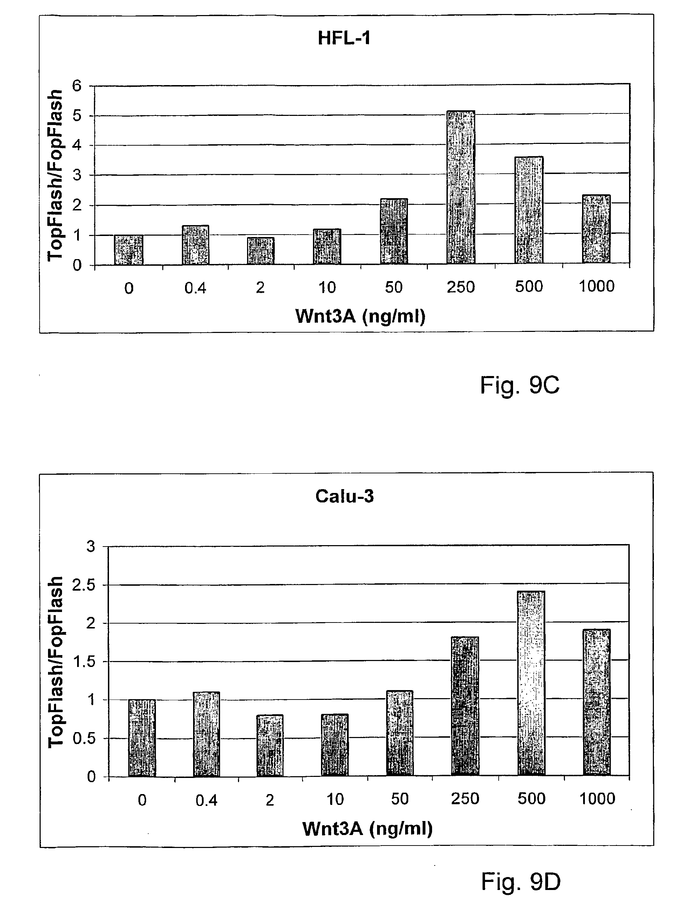

In order to analyze if Wnt3A is able to activate the Wnt-signal transduction pathway in humans we stimulated three different cell-lines from human origin with Wnt3A and measured the response with the TopFlash/FopFlash reporter assay. The three human lung derived cell-lines analyzed were; the lung epithelial carcinoma cell-line A549 (figure 9B), the normal lung fibroblast line HFL-1 (figure 9C) and the epithelial adenocarcinoma cell line calu-3 (Figure 9D). The results revealed that all three cell-lines respond to Wnt3A in a dosage dependent manner indicating that human and mouse lung cells are able to receive and transduce the Wnt-signal upon stimulation with Wnt3A.

### Expression profiling demonstrates that Wnt3A inhibits the differentiation of foetal lung explants.

To further investigate the effect of Wnt3A inhibition on lung differentiation we analyzed the expression profiles of Wnt3A treated lung explants with microarray analysis using the Affymetrix chip set MOE430AB, containing the complete mouse transcriptome. The lung explants, per condition 10 complete foetal lungs isolated from E12.5 embryos, were treated for 4 hours with 0 and 250 ng/ml Wnt3A (Wnt0/4hr and Wnt250/4hr respectively), and for 24 hours with 0, 10, 50 and 250 ng/ml Wnt3A (Wnt0/24hr, Wnt10/24hr, Wnt50/24hr, Wnt250/24hr, respectively). The 60 lung explants were isolated together from 6 pregnant Swiss mice and randomly distributed over the 6 different conditions. After the indicated time period, total RNA was isolated which was processed for the synthesis of a probe for hybridization onto the MOE-430AB chip sets. The results show a strong effect of Wnt3A upon gene expression in the lung explants. A rapid response was observed when the lungs were treated for 4 hours with 250 ng/ml Wnt3A. A large number of genes were differentially expressed more than two fold between the Wnt250/4hr group and Wnt0/4hr group ,i.e. 446 up regulated genes and 1165 down regulated genes. In accordance to the expectations, many known Wnt responsive genes were found in the up regulated gene pool, again showing that the Wnt-pathway was activated in these lung explants.

In the 24 hour treated explants a less pronounced effect was observed. In the 10 ng/ml wnt3a treated group 209 genes were up regulated and 223 genes were down regulated more than 2 fold. In the 50 ng/ml treated group 95 and 92 genes were up regulated and down regulated respectively. In the 250 ng/ml treated group 195 and 152 genes were up and down regulated more than two fold, respectively. However it should be noted that a concentration as low as 10 ng/ml Wnt3A is capable of inducing significant changes in gene expression. There is a strong correlation in the behavior of the genes in the 24 hour Wnt3A treated groups. Genes up- (or down) regulated in one of these groups were also up- (or down) regulated in the other two groups. Interestingly, there exists an opposite correlation between the genes up- or down regulated in the 4 hour treated group compared to the genes in the 24 hour treated group. With other words, genes up regulated in the 4 hours treated group were down regulated in the 24 hour treated group and visa versa.

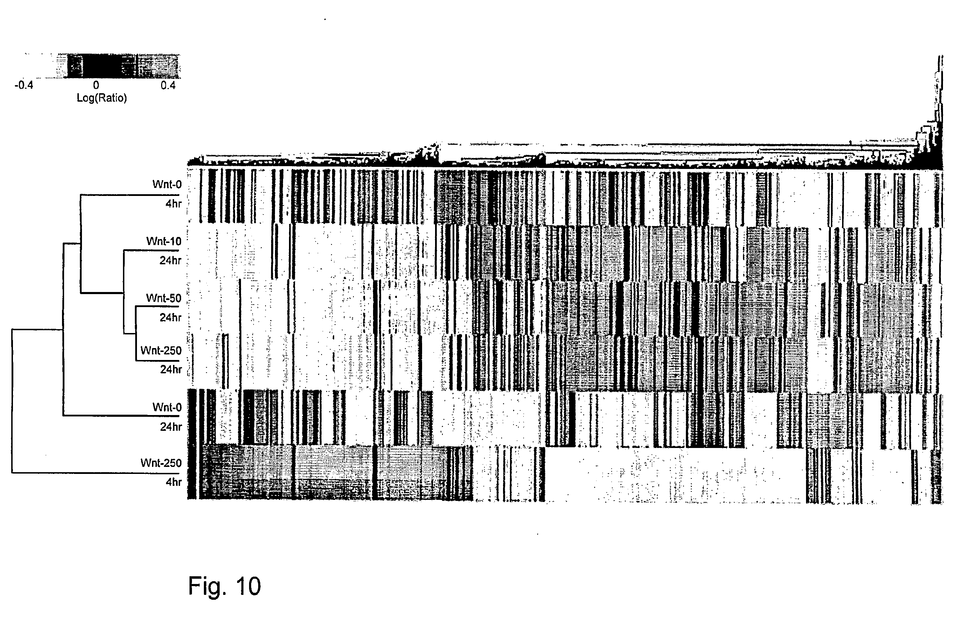

Cluster analysis of the expression profiles of genes significantly different in one of the 6 groups (4500 genes), in which genes with a comparable expression level are grouped, clearly shows that the three groups treated with 10 , 50 and 250 ng/ml Wnt3A during 24 hours, respond in a similar way (Figure 10) Importantly, the cluster analysis of 4500 significant changed genes (p< 0.000001) shows that the expression profiles of these three 24 hour Wnt3A treated lungs are strongly related to the 4 hour untreated lungs and these four conditions resemble each other more than the 24 hour untreated lungs. This shows that the transcriptome of 24 hour cultured lungs remains younger when cultured in the presence of Wnt3A. In other words Wnt3A inhibits at least partially the differentiation of foetal lung tissue.

The cluster analysis also shows that the 4 hour Wnt3A treated group has the most different expression profile of all 6 groups. Furthermore, it shows that this profile is for the majority of genes (circa 75%) opposite from the profiles of the 24 hour Wnt3A treated groups. Apparently, a strong feedback mechanism occurs upon Wnt3A stimulation after 24 hours. A mechanism which ultimately results in the inhibition of the differentiation and development of the lung.

### Conclusions

In this example we have shown that the addition of Wnt3A induces the nuclear accumulation of beta-catenin in primordial lung tissue; the hallmark of wnt-signal transduction. In addition we show that Wnt3A administration induces the expression of a wnt-reporter construct transfected in foetal lung tissue at least between 10 and 1000 ng/ml Wnt3A. The primordial lung thus has all the necessary components of the wnt-signal transduction pathway to recognize the Wnt3a polypeptide and to transduce its signal intracellular.

The addition of Wnt3a has a profound effect upon foetal lung development. Differentiation, cell-proliferation and apoptosis are greatly altered upon addition of Wnt3a to the culture medium. The effective concentration depends on the cell-type and the cellular function.

Using morphological criteria, marker gene analysis and microarray analysis we show that differentiation of the primordial lung epithelium is effectively inhibited between a concentration of 10 and 2000 ng/ml Wnt3A with an optimum between 50 and 500 ng/ml.

Proliferation of the bronchial and alveolar epithelium is markedly enhanced between 30 and 500 ng Wnt3a/ml culture medium, whereas at concentrations above 2000ng/ml proliferation is inhibited.

Apoptosis in the mesenchym is induced at concentrations higher than 100 ng/ml, whereas apoptosis in the epithelium is induced at concentrations higher than 1000 ng/ml. The mesenchym is thus much more sensitive for Wnt3A induced apoptosis than the epithelium.

The effects of Wnt3a upon differentiation, proliferation and apoptosis are strongly dependent upon cell-type and dosage. Our results show that at certain concentrations Wnt3a can, at the same time, inhibit alveolar differentiation, enhance epithelial proliferation and induce apoptosis in mesenchymal cells. The range in which all these three events occur in the above described hanging drop culture system is between 100 and 1000 ng Wnt3a/ml culture medium. The addition of Wnt3a is therefore suitable for selectively culturing and expanding in total number the undifferentiated stem cell population of the lung and for selectively inducing apoptosis in a mesenchymal cell population.

In addition we have shown that Wnt3A not only inhibits differentiation, it also restricts differentiation along the proximal-distal axis of the lung. This biological effect is also dosage dependent upon the concentration Wnt3A. As a rule of thumb, the higher the concentration of Wnt3A the more the differentiation is restricted to tissue types present in the proximal part of the lung, i.e. the tracheal epithelium with surrounding mesenchym and cartilage. The concentration range in which this biological effect took place is in the range of 10-1000 ng/ml. Between 10 and 250 ng/ml Wnt3A differentiation of distal epithelium was suppressed, whereas between 250 and 1000 ng/ml Wnt3a differentiation became primarily restricted to proximal lung tissue, such as bronchial epithelium, tracheal epithelium and cartilage. The addition of a certain concentration of Wnt3A polypeptide is thus useful for selectively differentiating primordial lung cells into proximal lung tissue. This tissue on its turn is then suitable for to treating diseases or injuries of the upper airways.

### Legends to the figures

**Figure 1A-1H**: Lung explant culture and subsequent immuno-histological analysis after five days, without the addition (fig 1A-D and 1I+J) and with the addition of Wnt3a (1000 ng/ml, fig 1 E-H and 1 K+L). Note the arrest of alveolar differentiation , the primordial character of the epithelium and the massive induction of apoptosis in the mesenchym in the presence of Wnt3a five days after the start of the culture.

**Figure 1I-1L** **:** Immunohistochemical analysis of employing PCNA as a marker for proliferation. Figure 1I+J; PCNA staining five days after the start of the culture without the addition of Wnt3a. Figure 1K+L; PCNA staining after five days of culture in the presence of Wnt3a (1000 ng/ml). Note the strong reduction in proliferation of the mesenchym versus the epithelium in the presence of Wnt3a.

**Figure 2A-2R****:** Lung explant culture and subsequent histological analysis 2 days after the start of the culture in the presence of Wnt3a at a concentration of 0 ng/ml (2A-C); 500 ng/ml (2D-F); 750 ng/ml (2G-I); 1000 ng/ml (2J-L); 2000 ng/ml (2M-O) and 4000 ng/ml (2P-R).

Note the increasing number of apoptotic cells in the mesenchym above 500 ng/ml Wnt3a and the induction of apoptosis in the epithelium above 2000 ng/ml Wnt3a.

**Figure 2S-2AD****.:** Immunohistochemical analysis of employing PCNA as a marker for proliferation 2 days after the start of the culture in the presence of Wnt3a at a concentration of 0 ng/ml (2S+T); 500 ng/ml (2U+V); 750 ng/ml (2W+X); 1000 ng/ml (2Y+Z); 2000 ng/ml (2AA+AB) and 4000 ng/ml (2AC+AD).

Note the elevated PCNA staining, compared to the control, in the bronchial epithelium in the prensence of 500 and 750 ng/ml Wnt3a and the absence of proliferation at concentrations above 2000 ng/ml.

**Figure 3****:** Proliferation in foetal lung explants cultured for 1 day with addition of 0ng/ml, 30 ng/ml, 125ng/ml and 250ng/ml Wnt3A. The number of all cells, and mesenchyme cells, were counted after immunuhistochemical staining against phospho histon H3 (ser10).

**Figure 4 A-O**: Lung explant culture and subsequent histological analysis 4 days after the start of the culture in the presence of Wnt3a at a concentration of 0 ng/ml (4A-C); 4 ng/ml (4D-F); 20 ng/ml (4G-I); 100 ng/ml (4J-L); and 500 ng/ml (4M-O). Note the increasing number of apoptotic cells in the mesenchym between above 100 ng/ml Wnt3a and the undifferentiated character of the epithelium four days after culture in the presence of 500 ng/ml Wnt3a (4M-O).

**Figure 5 A-H** **: Induction of apoptosis by Wnt3A.**

The TUNEL assay was performed on histological sections from Foetal lung explants, cultured for 4 days in the presence of Wnt3a at a concentration of 0 ng/ml (5A); 0.4 ng/ml (5B); 2 ng/ml (5C); 10 ng/ml (5D); 50 ng/ml (5E); 250 ng/ml (5F); 500 ng/ml (5G) and 1000 ng/ml (5H). Note the general increase in apoptotic cells (brown), in the mesenchyme above 100 ng/ml Wnt3A and the massive induction of apoptosis in the mesenchyme in contrast to the unaffected epithelium at 1000 ng/ml, illustrating the difference in sensitivity for Wnt3A induced apoptosis between mesenchyme and epithelium.

**Figure 6 A-H****: Effect of Wnt3A on proliferation in mesenchyme and epithelium.**

The incorporation of BrdU in newly synthesized DNA was analyzed by immunostaining of histological sections from Foetal lung explants, cultured for 4 days in the presence of Wnt3a at a concentration of 0 ng/ml (6A); 0.4 ng/ml (6B); 2 ng/ml (6C); 10 ng/ml (6D); 50 ng/ml (6E); 250 ng/ml (6F); 500 ng/ml (6G) and 1000 ng/ml (6H).

**Figure 7****: The effect of Wnt3A on the final lung differentiation.**

The lung explants were cultured for eight days in the presence of 0ng/ml (7A +B), 10ng/m (7C), 50ng/ml (7D), 250 ng/ml (7E) and 500 ng/ml Wnt3A (7F). After embedding, histological sections were stained with an tubulin-IV antibody to mark ciliated bronchial epithelium. In addition, the sections were counterstained with Haematoxylin and Alcian Blue which stained the mucus secreted from the bronchial epithelium and the cartilage associated with the tracheal epithelium. Note the abundance of bronchial epithelium, mucus and cartilage in the 500 ng/ml Wnt3A (7F) treated sample. In addition, note the loss of flattened epithelium (alveolar type-I cells) with increasing dosage of Wnt3A.

**Figure 8****: Nuclear translocation of beta-catenin by Wnt3A in foetal lung explants.**

The translocation of beta-catenin was determined using immuno-histological analysis of histological sections from Foetal lung explants (E12.5), cultured for 2 days in the presence of Wnt3a at a concentration of 0 ng/ml (8A); 10 ng/ml (8B); 50 ng/ml (8C); 250 ng/ml (8D); 500 ng/ml (8E) and 1000 ng/ml (8F). Note the strong induction of nuclear beta-catenin in the mesenchyme flanking the epithelium of the growing lung buds at concentrations of 250 ng/ml (8D) and 500 ng/ml Wnt3A (8E).

**Figure 9 A-D****: Induction of the Wnt-signaling pathway by Wnt3A in human and mouse lung cells.**

The induction of the Wnt-signaling pathway was measured by performing the Top Flah/Fop Flash reporter assay on primary lung fibroblasts isolated from a E12.5 foetal lung (9A), the adult human epithelial cell-line A549 (9B), the foetal human fibroblast cell-line HFL-1 (9C), and the adult human epithelial cell-line calu-3 (9D).

Note the strong induction of the Wnt-signaling pathway in all three human cell-lines, indicating that Wnt3A like in mouse is able to induce the Wnt-pathway in humans.

### Legend to figure 10:

Cluster analysis and the relationship of the expression profiles of 4500 genes significantly changed (p< 10⁻⁶) in expression in one of the six foetal lung explant cultures subjected to 0 ng/ml Wnt3A for 4 hours (0Wnt/4hr); 250 ng/ml Wnt3A for 4 hours (250Wnt/4hr); 0 ng/ml Wnt3A for 24 hours (0Wnt/24hr); 10 ng/ml Wnt3A for 24 hours (10Wnt/24hr); 50ngWnt3A for 24 hours (50Wnt/24hr); 250 ng Wnt3A for 24 hours (250Wnt/24hr). The expression levels of the 4500 genes are placed in six rows in which the expression level of each gene relatively to its mean expression level observed in all six conditions is represented by a coloured bar. A green bar corresponds to gene expressed below its mean expression level and a red bar correspond to genes expressed above its mean expression level.

Note the common expression profiles of the foetal lung cultures treated for 24 hours with 10 ng/ml, 50 ng/ml and 250 ng/ml Wnt3A and their closer resemblance with the expression profile of younger untreated lung cultures (0Wnt/4hr) than with the expression profile of untreated lung cultures of the same age (0Wnt/24hr), indicating that Wnt3A treatment inhibits lung differentiation and development.

### References

Behrens J, von Kries JP, Kuhl M, Bruhn L, Wedlich D, Grosschedl R and Birchmeier W. 1996. Functional interaction of beta-catenin with the transcription factor LEF-1. Nature, 382:638-42.
Bhanot P, Brink M, Samos CH, Hsieh JC, Wang Y, Macke JP, Andrew D, Cabilly et al :U.S. Pat. No. 4,816,567
Nathans J and Nusse R. 1996. A new member of the frizzled family from Drosophila functions as a Wingless receptor. Nature, 382:225-30.
Bienz M, Clevers H. Linking colorectal cancer to Wnt signaling. Cell. 2000 Oct 13;103(2):311-20.
Brown JD, Hallagan SE, McGrew LL, Miller JR and Moo RT. 2000. The maternal Xenopus beta-catenin signaling pathway, activated by frizzled homologs, induces goosecoid in a cell non-autonomous manner. Dev Growth Differ, 42:347-57, 2000.
Clackson T, Hoogenboom HR, Griffiths AD, Winter G. Making antibody fragments using phage display libraries.Nature. 1991 Aug 15;352(6336):624-8.
Hinck L, Nelson WJ and Papkoff J. 1994. Wnt-1 modulates cell-cell adhesion in mammalian cells by stabilizing beta-catenin binding to the cell adhesion protein cadherin. J Cell Biol, 124:729-41.
Kielman, M.F., Rindapää, M., Gaspar, C., van Poppel N., Breukel, C., van Leeuwen, S., Taketo, M.M., Roberts, S., Smits, R., Fodde, R. 2002. Apc modulates embryonic stem-cell differentiation by controlling the dosage of Beta catenin signaling. Nature Genetics 32:594-605
Kohler G, Milstein C. Continuous cultures of fused cells secreting antibody of predefined specificity. Nature. 1975 Aug 7;256(5517):495-7.
Korinek V, Barker N, Willert K, Molenaar M, Roose J, Wagenaar G, Markman M, Lamers W, Destree O and Clevers H. 1998. Two members of the Tcf family implicated in Wnt/beta-catenin signaling during embryogenesis in the mouse. Mol Cell Biol, 18:1248-56.
Lee SM, Tole S, Grove E and McMahon AP. 2000. A local Wnt-3a signal is required for development of the mammalian hippocampus. Development, 127:457-67.
Marks JD, Hoogenboom HR, Bonnert TP, McCafferty J, Griffiths AD, Winter G. By-passing immunization. Human antibodies from V-gene libraries displayed on phage. J Mol Biol. 1991 Dec 5;222(3):581-97.
McMahon AP, Gavin BJ, Parr B, Bradley A and McMahon JA.1992. The Wnt family of cell signalling molecules in postimplantation development of the mouse. Ciba Found Symp, 165:199-212; discussion 212-8.
Matthews, W., Austin, T.W.,1997 Uses of Wnt polypeptides US patent 6,159,462
Morrison SL, Johnson MJ, Herzenberg LA, Oi VT. Chimeric human antibody molecules: mouse antigen-binding domains with human constant region domains. Proc Natl Acad Sci U S A. 1984 Nov;81(21):6851-6855.
Nusse R and Varmus HE. 1992. Wnt genes. Cell, 69:1073-87
Reya T., Duncan, A.W.; Ailles, L.; Domen, J.; Scherer, D.C.; Willert, K.; Hintz, L.; Nusse, R.; Weissmann, I.L.: A role for Wnt signalling in self-renewal of haematopoietic stem cells Nature, May 2003, 409-414
Stark K, Vainio S, Vassileva G and McMahon AP.1994. Epithelial transformation of metanephric mesenchyme in the developing kidney regulated by Wnt-4. Nature, 372:679-83.
Willert K and Nusse R. 1998. Beta-catenin: a key mediator of Wnt signaling. Curr Opin Genet Dev, 8:95-102.
Willert K, Brown JD, Danenberg E, Duncan AW, Weissman IL, Reya T, Yates JR 3rd, Nusse R. Wnt proteins are lipid-modified and can act as stem cell growth factors. Nature. 2003 May 22;423(6938):448-52.
Wodarz A and Nusse R. 1998. Mechanisms of Wnt signaling in development. Annu Rev Cell Dev Biol, 14:59-88.

## Claims

1. An in vitro method for inhibiting differentiation of a lung stem cell, comprising providing said stem cell with Wnt3a or with a biologically active Wnt3a variant having at least 90% amino acid sequence identity with Wnt3a.

2. An in vitro method for inducing a lung stem cell to proliferate by inhibiting differentiation of said stem cell, comprising providing said stem cell with Wnt3a or with a biologically active Wnt3a variant having at least 90% amino acid sequence identity with Wnt3a.

3. A method according to any of claims 1 to 2, wherein said stem cell is provided with Wnt3a or with a biologically active Wnt3a variant having at least 90% amino acid sequence identity with Wnt3a in an amount of at least 1 ng and at most 2000 ng per ml tissue culture fluid.

4. A method according to any of claims 1-3 wherein the amount of Wnt3a or biologically active Wnt3a variant having at least 90% amino acid sequence identity with Wnt3a is from at least 10 to at most 1500 ng per ml tissue culture fluid.

5. A method according to any of claims 1-4 wherein the amount of Wnt3a or biologically active Wnt3a variant having at least 90% amino acid sequence identity with Wnt3a is from at least 20 to at most 1000 ng per ml tissue culture fluid.

6. A method according to any of claims 1-5 wherein the amount of Wnt3a or biologically active Wnt3a variant having at least 90% amino acid sequence identity with Wnt3a is from at least 30 to at most 500 ng per ml tissue culture fluid.

7. An in vitro method for enriching a population of lung stem cells in a population of mesenchymal cells comprising said lung stem cells, by inducing apoptosis in the mesenchymal cells by providing said mesenchymal cells with Wnt3a or with a biologically active Wnt3a variant having at least 90% amino acid sequence identity with Wnt3a.

8. A method according to claim 7, wherein said mesenchymal stem cell is provided with Wnt3a or with a biologically active Wnt3a variant having at least 90% amino acid sequence identity with Wnt3a in an amount of at least 50 ng and at most 1500 ng per ml tissue culture fluid.

9. A method according to claim 7 or 8, wherein said mesenchymal stem cell is provided with Wnt3a or with a biologically active Wnt3a variant having at least 90% amino acid sequence identity with Wnt3a in an amount of at least 100 ng and at most 1000 ng per ml tissue culture fluid.

10. An in vitro method for providing a plurality of lung stem cells from a population of mesenchymal cells and lung stem cells, comprising providing said mesenchymal cells with an apoptosis inducing amount of Wnt3a or with a biologically active Wnt3a variant having at least 90% amino acid sequence identity with Wnt3a and providing said lung stem cells with a differentiation inhibiting amount of Wnt3a or a biologically active Wnt3a variant having at least 90% amino acid sequence identity with Wnt3a.

11. A method according to claim 10 wherein said Wnt3a or biologically active Wnt3a variant having at least 90% amino acid sequence identity with Wnt3a is provided in an amount of at least 50 ng and at most 1500 ng per ml tissue culture fluid.

12. A method according to claim 10 or 11 wherein differentiation of a distal lung cell type is at least partly inhibited by administering 10- 250 ng of Wnt3a or biologically active Wnt3a variant having at least 90% amino acid sequence identity with Wnt3a /ml tissue culture fluid.

13. A method according to claim 12, wherein said distal lung cell type is a type I or type II alveolar cell.

14. A method according to claim 10 or 11, wherein proliferation and differentiation of an upper airway cell type is selectively increased by administering 10-750 ng of Wnt3a or a biologically active Wnt3a variant having at least 90% amino acid sequence identity with Wnt3a /ml tissue culture fluid.

15. A method according to claim 14 wherein said upper airway cell type is a tracheal and/or bronchial epithelial cell.

16. Use of Wnt3a or a biologically active Wnt3a variant having at least 90% amino acid sequence identity with Wnt3a for the preparation of a medicament for treatment of lung failure, lung surfactant-deficiency, emphysema, chronic respiratory distress and/or lung cancer.

## Patentansprüche

1. In-vitro-Verfahren zur Hemmung der Differenzierung einer Lungenstammzelle, umfassend das Versehen der Stammzelle mit Wnt3a oder mit einer biologisch aktiven Wnt3a-Variante, die wenigstens 90% Aminosäuresequenzidentität mit Wnt3a aufweist.

2. In-vitro-Verfahren zur Induktion der Vermehrung einer Lungenstammzelle durch Hemmung der Differenzierung dieser Stammzelle, umfassend das Versehen der Stammzelle mit Wnt3a oder mit einer biologisch aktiven Wnt3a-Variante, die wenigstens 90% Aminosäuresequenzidentität mit Wnt3a aufweist.

3. Verfahren gemäß einem der Ansprüche 1 bis 2, wobei die Stammzelle mit Wnt3a oder mit einer biologisch aktiven Wnt3a-Variante, die wenigstens 90% Aminosäuresequenzidentität mit Wnt3a aufweist, in einer Menge von wenigstens 1 ng und höchstens 2000 ng pro ml Gewebekulturflüssigkeit versehen wird.

4. Verfahren gemäß einem der Ansprüche 1 bis 3, wobei die Menge des Wnt3a oder der biologisch aktiven Wnt3a-Variante, die wenigstens 90% Aminosäuresequenzidentität mit Wnt3a aufweist, wenigstens 10 bis höchstens 1500 ng pro ml Gewebekulturflüssigkeit beträgt.

5. Verfahren gemäß einem der Ansprüche 1 bis 4, wobei die Menge des Wnt3a oder der biologisch aktiven Wnt3a-Variante, die wenigstens 90% Aminosäuresequenzidentität mit Wnt3a aufweist, wenigstens 20 bis höchstens 1000 ng pro ml Gewebekulturflüssigkeit beträgt.

6. Verfahren gemäß einem der Ansprüche 1 bis 5, wobei die Menge des Wnt3a oder der biologisch aktiven Wnt3a-Variante, die wenigstens 90% Aminosäuresequenzidentität mit Vlfnt3a aufweist, wenigstens 30 bis höchstens 500 ng pro ml Gewebekulturflüssigkeit beträgt.

7. In-vitro-Verfahren zur Anreicherung einer Population von Lungenstammzellen in einer Population von Mesenchymzellen, die diese Lungenstammzellen umfasst, durch Induzieren einer Apoptose in den Mesenchymzellen durch Versehen der Mesenchymzellen mit Wnt3a oder mit einer biologisch aktiven Wnt3a-Variante, die wenigstens 90% Aminosäuresequenzidentität mit Wnt3a aufweist.

8. Verfahren gemäß Anspruch 7, wobei die mesenchymale Stammzelle mit Wnt3a oder mit einer biologisch aktiven Wnt3a-Variante, die wenigstens 90% Aminosäuresequenzidentität mit Wnt3a aufweist, in einer Menge von wenigstens 50 ng und höchstens 1500 ng pro ml Gewebekulturflüssigkeit versehen wird.

9. Verfahren gemäß Anspruch 7 oder 8, wobei die mesenchymale Stammzelle mit Wnt3a oder mit einer biologisch aktiven Wnt3a-Variante, die wenigstens 90% Aminosäuresequenzidentität mit Wnt3a aufweist, in einer Menge von wenigstens 100 ng und höchstens 1000 ng pro ml Gewebekulturflüssigkeit versehen wird.

10. In-vitro-Verfahren zur Gewinnung einer Vielzahl von Lungenstammzellen aus einer Population von Mesenchymzellen und Lungenstammzellen, umfassend das Versehen der Mesenchymzellen mit einer Apoptoseinduzierenden Menge an Wnt3a oder einer biologisch aktiven Wnt3a-Variante, die wenigstens 90% Aminosäuresequenzidentität mit Wnt3a aufweist, und Versehen der Lungenstammzellen mit einer die Differenzierung hemmenden Menge an Wnt3a oder einer biologisch aktiven Wnt3a-Variante, die wenigstens 90% Aminosäuresequenzidentität mit Wnt3a aufweist.

11. Verfahren gemäß Anspruch 10, wobei das Wnt3a oder die biologisch aktive Wnt3a-Variante, die wenigstens 90% Aminosäuresequenzidentität mit Wnt3a aufweist, in einer Menge von wenigstens 50 ng und höchstens 1500 ng pro ml Gewebekulturflüssigkeit bereitgestellt wird.

12. Verfahren gemäß Anspruch 10 oder 11, wobei die Differenzierung eines distalen Lungenzelltyps durch Verabreichen von 10-250 ng Wnt3a oder einer biologisch aktiven Wnt3a-Variante, die wenigstens 90% Aminosäuresequenzidentität mit Wnt3a aufweist, pro ml Gewebekulturflüssigkeit wenigstens zum Teil gehemmt wird.

13. Verfahren gemäß Anspruch 12, wobei es sich bei dem distalen Lungenzelltyp um eine Alveolarepithelzelle Typ I oder Typ II handelt.

14. Verfahren gemäß Anspruch 10 oder 11, wobei die Vermehrung und Differenzierung eines Zelltyps der oberen Atemwege durch Verabreichen von 10-750 ng Wnt3a oder einer biologisch aktiven Wnt3a-Variante, die wenigstens 90% Aminosäuresequenzidentität mit Wnt3a aufweist, pro ml Gewebekulturflüssigkeit selektiv erhöht wird.

15. Verfahren gemäß Anspruch 14, wobei es sich bei dem Zelltyp der oberen Atemwege um eine Epithelzelle der Luftröhre und/oder der Bronchien handelt.

16. Verwendung von Wnt3a oder einer biologisch aktiven Wnt3a-Variante, die wenigstens 90% Aminosäuresequenzidentität mit Wnt3a aufweist, zur Herstellung eines Medikaments zur Behandlung von Lungenversagen, Lungensurfactantmangel, Emphysem, chronischer Atemnot und/oder Lungenkrebs.

## Revendications

1. Procédé *in vitro* pour inhiber la différenciation d'une cellule souche pulmonaire, qui consiste à fournir à ladite cellule souche Wnt3a ou un variant de Wnt3a biologiquement actif présentant au moins 90 % d'identité de séquence d'acides aminés avec Wnt3a.

2. Procédé *in vitro* pour induire la prolifération d'une cellule souche pulmonaire en inhibant la différenciation de ladite cellule souche, qui consiste à fournir à ladite cellule souche Wnt3a ou un variant de Wnt3a biologiquement actif présentant au moins 90 % d'identité de séquence d'acides aminés avec Wnt3a.

3. Procédé selon l'une quelconque des revendications 1 à 2, dans lequel on fournit à ladite cellule souche Wnt3a ou un variant de Wnt3a biologiquement actif, présentant au moins 90 % d'identité de séquence d'acides aminés avec Vnt3a en une quantité d'au moins 1 ng et au maximum de 2000 ng par ml de liquide de culture tissulaire.

4. Procédé selon l'une quelconque des revendications 1-3, dans lequel la quantité de Wnt3a ou d'un variant de Wnt3a biologiquement actif présentant au moins 90 % d'identité de séquence d'acides aminés avec Wnt3a est comprise entre au moins 10 et au maximum 1500 ng par ml de liquide de culture tissulaire.

5. Procédé selon l'une quelconque des revendications 1-4, dans lequel la quantité de Wnt3a ou d'un variant de Wnt3a biologiquement actif présentant au moins 90 % d'identité de séquence d'acides aminés avec Wnt3a est comprise entre au moins 20 et au maximum 1000 ng par ml de liquide de culture tissulaire.

6. Procédé selon l'une quelconque des revendications 1-5, dans lequel la quantité de Wnt3a ou d'un variant de Wnt3a biologiquement actif présentant au moins 90 % d'identité de séquence d'acides aminés avec Wnt3a est comprise entre au moins 30 et au maximum 500 ng par ml de liquide de culture tissulaire.

7. Procédé *in vitro* pour enrichir une population de cellules souches pulmonaires dans une population de cellules mésenchymateuses comprenait lesdites cellules souches pulmonaires, par une induction de l'apoptose dans les cellules mésenchymateuses en fournissant auxdites cellules mésenchymateuses Wnt3a ou un variant de Wnt3a biologiquement actif présentant au moins 90 % d'identité de séquence d'acides aminés avec Wnt3a.

8. Procédé selon la revendication 7, dans lequel on fournit à ladite cellule souche mésenchymateuse Wnt3a ou un variant de Wnt3a biologiquement actif présentant au moins 90 % d'identité de séquence d'acides aminés avec Wnt3a en une quantité d'au moins 50 ng et au maximum de 1500 ng par ml de liquide de culture tissulaire.

9. Procédé selon la revendication 7 ou 8, dans lequel on fournit à ladite cellule souche mésenchymateuse Wnt3a ou un variant de Wnt3a biologiquement actif présentant au moins 90 % d'identité de séquence d'acides aminés avec Wnt3a en une quantité d'au moins 100 ng et au maximum de 1000 ng par ml de liquide de culture tissulaire.

10. Procédé *in vitro* pour obtenir une pluralité de cellules souches pulmonaires à partir d'une population de cellules mésenchymateuses et de cellules souches pulmonaires, qui consiste à fournir auxdites cellules mésenchymateuses une quantité induisant l'apoptose de Wnt3a ou d'un variant de Wnt3a biologiquement actif présentant au moins 90 % d'identité de séquence d'acides aminés avec Wnt3a, et à fournir auxdites cellules souches pulmonaires une quantité inhibant la différenciation de Wnt3a ou d'un variant de Wnt3a biologiquement actif présentant au moins 90 % d'identité de séquence d'acides aminés avec Wnt3a.

11. Procédé selon la revendication 10, dans lequel ledit Wnt3a ou variant de Wnt3a biologiquement actif présentant au moins 90 % d'identité de séquence d'acides aminés avec Wnt3a est fourni en une quantité d'au moins 50 ng et au maximum de 1500 ng par ml de liquide de culture tissulaire.

12. Procédé selon la revendication 10 ou 11, dans lequel la différenciation d'un type de cellule pulmonaire distale est au moins partiellement inhibée en administrant 10-250 ng de Wnt3a ou d'un variant de Wnt3a biologiquement actif présentant au moins 90 % d'identité de séquence d'acides aminés avec Wnt3a/ml de liquide de culture tissulaire.

13. Procédé selon la revendication 12, dans lequel ledit type de cellule pulmonaire distale est une cellule alvéolaire de type I ou de type II.

14. Procédé selon la revendication 10 ou 11, dans lequel la prolifération et la différenciation d'un type de cellule des voies aériennes supérieures est sélectivement augmentée en administrant 10-750 ng de Wnt3a ou d'un variant de Wnt3a biologiquement actif présentant au moins 90 % d'identité de séquence d'acides aminés avec Wnt3a/ml de liquide de culture tissulaire.

15. Procédé selon la revendication 14, dans lequel ledit type de cellule des voies aériennes supérieures est une cellule épithéliale trachéale et/ou bronchique.

16. Utilisation de Wnt3a ou d'un variant de Wnt3a biologiquement actif présentant au moins 90 % d'identité de séquence d'acides aminés avec Wnt3a pour la préparation d'un médicament destiné au traitement d'une insuffisance pulmonaire, d'un déficit en surfactant pulmonaire, de l'emphysème, d'une détresse respiratoire chronique et/ou du cancer du poumon.
